(19)

Europäisches
Patentamt
European
Patent Office
Office européen
des brevets

(11) **EP 4 386 091 A1**

(12) **EUROPEAN PATENT APPLICATION**

(43) Date of publication:
**19.06.2024 Bulletin 2024/25**

(21) Application number: **22213544.4**

(22) Date of filing: **14.12.2022**

(51) International Patent Classification (IPC):
***C12Q 1/689*** *(2018.01)*

(52) Cooperative Patent Classification (CPC):
**C12Q 1/689;** C12Q 2600/118; C12Q 2600/156

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **The BioArte Limited
MST 9012 Mosta (MT)**

(72) Inventors:
• **BIAZZO, Manuele
Qawra, SPB1257 (MT)**

• **PINZAUTI, David
50012 Bagno a Ripoli, Firenze (IT)**
• **D'AGUANNO, Maria
Gzira, GZR (MT)**
• **SCHRAMM, Laetita
Pieta, PTA1313 (MT)**

(74) Representative: **Ghirardi, Valeria et al
De Simone & Partners S.r.l.
Via Vincenzo Bellini, 20
00198 Roma (IT)**

<u>Remarks:</u>
The complete document including Reference
Table(s) and the Sequence Listing(s) can be
downloaded from the EPO website

(54) **A METHOD FOR TAXONOMIC CLASSIFICATION UP TO SPECIES LEVEL OF BACTERIA**

(57) The present invention relates to a method for identifying and optionally quantifying microorganisms in a sample wherein the method is capable of allowing species-level classification of the microorganism identified or quantified in the sample

EP 4 386 091 A1

**Description**

**TECHNICAL FIELD**

The present invention relates to a method for identifying and optionally quantifying microorganisms from their nucleic acids in a biological sample obtained from a subject wherein the method is capable of allowing species-level classification of the microorganism identified or quantified in the biological sample and to applications of the methods in different fields, in particular for identifying and optionally quantifying the microorganisms present in the gut microbiota or blood, or urine, or synovial fluid, or pulmonary wash samples. The method of the invention may also be applied as a non-invasive diagnosis tool for IBD.

**BACKGROUND ART**

The 16S rRNA sequence is the best single molecular marker for identifying bacteria, and with the development of high-throughput sequencing methods, 16S rRNA gene amplicon sequencing became a pivotal tool for the characterization of bacterial diversity and community structure of almost any environment (Janda et al. 2007, Pollock et al. 2018). Since the introduction of the MiSeq in 2011, Illumina instruments became the most widely used platforms for 16S rRNA gene sequencing (Pichler et al. 2018). They offer very low error rates but are limited in read length currently capable of reaching only $2 \times 300$ bp. In addition, the cost of Illumina devices is well over the budget of most research groups. As a result, instead of performing the sequencing runs in-house, it became common practice to ship DNA extracts or PCR products to sequencing centres. Furthermore, the results do not have the full information content of the 16S rRNA gene as the reads can cover only one, two, or at most three out of the nine variable regions. This means that Illumina by definition could not reach a valuable result with 16S rRNA gene amplicon sequencing, that will be used in clinical practise. Having to outsource the sequencing limits the possibility of optimizing the library preparation and run parameters for the needs of each research project while the short reads limit the taxonomic accuracy of the results (Johnson et al. 2019). A promising solution to this is Nanopore sequencing which has two crucial properties: long reads and low up-front cost. Firstly, it can sequence the full 16S rRNA gene retrieving the information content of all nine variable regions. Secondly, the cost of a MinION is a small fraction of that of any Illumina or other high-throughput sequencing system. This enables individual research groups to set up and optimise their own library preparation and sequencing workflows. In addition, it is a small, portable device allowing the processing of samples on site without the need of elaborate laboratory infrastructure. Consequently, there is growing interest of using Nanopore for 16S rRNA gene amplicon sequencing and recent publications demonstrated its success with a variety of samples for example freshwater (Urban et al. 2021), seawater and algal surfaces (Van der Loos et al. 2021), soil (Chavan et al. 2022, Li et al. 2022), surfaces in the international space station (Stahl-Rommel et al. 2021), animal faeces (Beale et al. 2022), urine (Zhang et al. 2022), buccal and rectal swabs (Ammer-Hermenau et al. 2021), ophthalmic samples (Omi et al. 2022), bronchoalveolar lavage fluid, blood, pleural and ascites fluid, cerebrospinal fluid, and wound secretions (Fu et al. 2022). The most important limitation of Nanopore sequencing is its comparatively high error rate. The benefit that the higher information content of the long reads provides for taxonomic classification is lost if the reads have low accuracy. In 2019, sequencing of mock communities of known composition led to the conclusion that species-level classification was not yet reliable with Nanopore (Winand et al. 2019). Later studies determined the sequencing accuracy to be 92.08% (Urban et al. 2021) and 93.17 - 95.97% (Delahaye and Nicolas 2021). Considering that the threshold to distinguish bacterial species based on their 16S rRNA sequence has traditionally been 97% similarity and later revised to be 98.65% similarity (Kim et al. 2014), it is expected that species-level taxonomic classification of Nanopore reads is problematic with such sequencing accuracy rates. However, the introduction of the kit 12 chemistry in late 2021 brought a substantial improvement with >99% accuracy becoming attainable (nanoporetech.com). This makes the question of whether, or in which cases, Nanopore should be adopted as the preferred sequencing technology instead of Illumina for 16S rRNA gene amplicon sequencing ever more relevant. To get a definitive answer, a comparison of Nanopore and Illumina sequencing results is needed that 1 includes samples of known composition such as a mock community, 2 includes real samples of high bacterial diversity, 3 compares the results on diversity, richness, and community structure instead of focusing only on specific taxa, such as potential pathogens, 4 assesses replicability, i.e. the differences between repeated analyses of the same sample, and 5 preferably uses the same bioinformatical tools and taxonomic reference database to process the Nanopore and Illumina data. Publications that include comparisons between Nanopore and Illumina 16S rRNA gene amplicon sequencing results are listed in **Table** 1. They fulfilled some of the above points; however, covering all of them in a single study using the current Nanopore sequencing chemistry with improved accuracy is missing.

**Table 1: Published studies comparing 16S rRNA gene amplicon sequencing with Illumina and Nanopore**

| Samples | Mock community included | Nanopore sequencing | Illumina sequencing | Difference in community structure | Difference in diversity | Difference in replicability | Reference |
|---|---|---|---|---|---|---|---|
| **2 faecal samples from mice** | No | V1-9, SQK-MAP006 | V3-4 | No difference except at species-level | Not compared | Not assessed | Shin et al. 2016 |
| **13 water samples** | Yes | V1-9, SQK-RAB204 | V4 | Focused on the detection of potential pathogens and faecal indicators | Not compared | Not assessed | Acharya et al. 2019 |
| **One mock community** | Yes | V 1-9, SQK-RAB201 | V1-3, V3-4, V4, V4-5, V4-6, V6, V8, V8-9 | Good results with both at genus-level | Not compared | Not assessed | Winand et al. 2019 |
| **6 tap water and biofilm samples** | Yes | V1-9, SQK-RAB204 or SQK-RAB201 | V3-4 | Only the 15 most abundant genera were considered | Not compared | Not assessed | Fujiyoshi et al. 2020 |
| **59 human nasal swabs** | No | V 1-9, SQK-RAB201 | V5-6 | Similar results | Similar diversity | Not assessed | Heikema et al. 2020 |
| **12 indoor dust samples** | No | V 1-9, SQK-LSK108 | V3-4 | Difference at genus- and species-level, but not at family-level | Not compared | Not assessed | Nygaard et al. 2020 |
| **50 human faecal samples** | No | V 1-9, SQK-16S024 | V3-4 | Good correlation at genus-level but not at species-level | Not compared | Not assessed | Wei et al. 2020 |
| **Only a mock community was included in the comparison with Illumina** | Yes | V1-9 and 16S-ITS-23S, SQK-PBK004 | V3-4 | Nanopore performed better at genus-level | Not compared | Only assessed for 16SITS-23S | Kinoshita et al. 2021 |
| **Corneal and conjunctival swabs from 2 patients** | No | V1-9, kit not specified | V4 | Similar results | Not compared | Not assessed | Low et al. 2021 |
| **6 human faecal samples** | Yes | V1-9 and V3-4, SQK-PBK004 | V3-4 | Only the 15 most abundant genera were considered | Not compared | Not assessed | Matsuo et al. 2021 |

(continued)

| Samples | Mock community included | Nanopore sequencing | Illumina sequencing | Difference in community structure | Difference in diversity | Difference in replicability | Reference |
|---|---|---|---|---|---|---|---|
| **8 human endometrial tissue samples** | No | V 1-9, SQK-RAB204 | 6 primer pairs pooled to cover V1-9 | Similar results | Not compared | Not assessed | Oberle et al. 2021 |
| **8 mock communities** | Yes | V 1-9, SQK-LSK108 | V1-2, V1-3, V3, V4 | Nanopore showed overall higher bias | Not compared | Not assessed | Park et al. 2021 |
| **31 surface swabs from intensive care units** | No | V 1-9, SQK-16S024 | V4 | Focused on the detection of potential pathogens | Not compared | Not assessed | De Siqueria et al. 2021 |
| **A mixture of DNA from 72 cheek skin swabs** | Yes | V1-9 and 16S-ITS-23S, SQK-LSK110 | V 1-3 | Similar results | Not compared | Good correlation between duplicates | Rozas et al. 2022 |
| **8 soil samples from 2 sites** | Yes | V1-9 and V3-4, SQK-LSK109 | V3-4 | Significant effect but not as strong as the differences between sites | Difference in richness estimates | Not assessed | Stevens et al. 2022 |

**Applicability of method as diagnostic tool for Inflammatory bowel disease** (IBD)

Inflammatory Bowel Disease (IBD) is a group of digestive disorders that are characterized by a chronic and relapsing inflammation of the gastrointestinal (GI) tract (Hanauer, S. B., 2016)). The two most common IBD subtypes are Crohn's disease (CD) and Ulcerative colitis (UC) (Hanauer, S. B., 2016). CD and UC can display very similar symptoms, making the diagnosis difficult, but they are two distinct pathologies which affect different regions of the gut and require specific treatments (Baumgart, D. C. & Sandborn, W. J. 2007). CD can affect any part of the digestive tract, from the mouth to the perianal region, whereas UC is principally restricted to the large intestine (Baumgart, D. C. & Sandborn, W. J. 2007, Yu, Y. R. & Rodriguez, J. R. 2017). Additionally, CD is characterized by a transmural and patchy inflammation, with inflamed areas separated from each other by healthy sections, while UC lesions are continuous and mainly affect the inner layer of the colon (Baumgart, D. C. & Sandborn, W. J. (2007), Yu, Y. R. & Rodriguez, J. R. 2017). It is estimated that about 6 million of people are currently living with IBD in the world, with the highest IBD rates reported in North America and Western Europe (Silangcruz, K. et al. 2021; Kaplan, G. G. 2015; Abdulla, M. & Mohammed, N. A 2022). Additionally, the prevalence and incidence of pediatric-onset IBD have been shown to continuously increase worldwide, with new cases emerging in countries that were, until now, spared by the disease (Kuenzig, M. E. et al. 2022). Although displaying similarities to the adult form, there is growing evidence suggesting that pediatric IBD (which represents up to 25% of newly diagnosed cases (Yu, Y. R. & Rodriguez, J. R. (2017) constitutes a distinct type of diseases that raises specific challenges (Sauer, C. G. & Kugathasan, S. 2010; Rosen, M. J., Dhawan, A. & Saeed, S. A. 2015). For example, the disease type, disease location, disease behavior, genetic risk factors and treatments differ from what is observed in the adult form of IBD (Yu, Y. R. & Rodriguez, J. R. 2017; Sauer, C. G. & Kugathasan, S. Pediatric 2010, Kelsen, J. & Baldassano, R. N. 2008). At the moment, the standard way to diagnose IBD principally relies on endoscopy and colonoscopy examination, as well as histologic analysis of biopsy samples (Rosen, M. J., Dhawan, A. & Saeed, S. A. 2015; Dragoni, G., Innocenti, T. & Galli, A. 2021). Those techniques are particularly invasive and performed under general anesthesia in children (Olbjorn, C., Småstuen, M. C. & Moen, A. E. F. 2022). Additionally, categorizing IBD patients into CD or UC is particularly challenging, as in young subjects, CD often solely involves the large intestine, making the differentiation to UC difficult (Olbjorn, C., Småstuen, M. C. & Moen, A. E. F. 2022). Diagnostic delays and/or misdiagnosis are frequent, and prevent access to effective early therapies, which can lead to irreversible complications (such as growth delay) in kids (Kelsen, J. & Baldassano, 2008; Rosen, M. J., Dhawan, A. & Saeed, S. A. 2015; Olbjorn, C., Småstuen, M. C. & Moen, A. E. F. 2022). The need for specific, sensitive, and non-invasive IBD markers has been intensively addressed in the scientific community. Indeed, although serologic and genetic markers are commercially available, it was shown that sizeable number of children suffering from IBD will have negative test results for these markers, of which the sensitivity is estimated between 65% to 75% (Rosen, M. J., Dhawan, A. & Saeed, S. A. 2015). Fecal calprotectin (neutrophil derived protein), currently considered as a reliable marker in IBD management, shows a strong sensitivity (98%) but only displays 68% specificity in children with suspected IBD (Rosen, M. J., Dhawan, A. & Saeed, S. A. 2015). Although the exact etiology of IBD remains unclear, multiple factors are involved in the pathogenesis, including an abnormal immune response, genetic factors, environmental components, and the host gut microbiome (Abdulla, M. & Mohammed, N. A 2022; Zhao, M., Gönczi, L., Lakatos, P. L. & Burisch, J. 2021). In the past decades, a growing interest in understanding the role of the intestinal microbiota in the pathogenesis of IBD has been observed. It was shown that the gut microbiota composition of IBD patients differ from control individuals, and it was notably associated with a decreased richness and evenness, compared to non-IBD subjects (Sila, S. et al. 2020). At the phylum level, studies generally report a decreased relative abundance of Firmicutes in IBD patients, while Proteobacteria relative abundance is shown to be higher in comparison to healthy individuals (Sila, S. et al. 2020). Additionally, increased relative abundance of hydrogen sulfide producers (including *Veillonella, Streptococcus, Fusobacterium, Peptococcus)* and decreased relative abundance of butyrate producers *(Ruminococcus, Blautia, Roseburia, Eubacterium rectale)* have been reported in pediatric treatment naive CD patients (Mottawea, W. et al. 2016). Finally, in the past, Gevers et al., reported a relevant dysbiosis index (defined as the log of [total abundance in organisms increased in CD]/[total abundance of organisms decreased in CD]) for treatment naive pediatric CD patients (Gevers, D. et al. 2014). However, the current published studies profiling IBD patients' gut microbiota are only partially sequencing the 16S rRNA gene using Illumina technology (Gevers, D. et al. 2014). Focusing on one of the nine hypervariable regions contained in the 16S rRNA gene does not provide sufficient information to claim accurate species identification.

Therefore there is still the need to reach species level in the analysis of microorganisms from nucleic acids and to provide an index based on species level.

### SUMMARY OF THE INVENTION

Using faecal samples collected from six individuals and a mock community sample, inventors compared 16S rRNA gene amplicon sequencing results from Nanopore and Illumina to assess their ability to distinguishing samples based on the bacterial community structure at species- and higher taxonomic levels, deliver replicable results and good coverage of species richness, and to characterize their differences in taxonomic bias. Each sample was sequenced in three technical replicates with both sequencing platforms. The reads were processed with an in house custom data analysis pipeline (now on referred as MiTRA).

The present inventors found out that by adding to the current standard nanopore pipelines some innovative steps, in particular : change in primers, change in PCR parameters, change in library preparation, change in software analyzing results, customization of the report it was possible to mitigate the taxonomic bias of the results, obtain data quality sufficient for reliable species-level classification, and generate reports showing species-level taxonomic resolution.

Further, inventors found out a MIDIBD (Microbiome Index for Dysbiosis IBD related) by calculating:

Log of [total abundance in selected species increased in IBD] over [total abundance of selected species decreased in IBD]

wherein the species increased are selected from hydrogen sulfide producers the Species decreased are selected from butyrate producing-bacterial species. This index is particularly significant for European samples.

This index has been developed by comparing 100 IBD samples (62 adults and 40 paediatric) with 100 (paediatric and adults healthy controls). Species were selected using indicator species analysis tools in R with p value less than 0.05.

The inventors found that the present index, when compared to reference value(s), allows to diagnose and/or prognose a Dysbiosis, IBD related, or IBD and/or to monitor the efficacy of a therapeutic treatment of a Dysbiosis IBD related or of IBD and/or to screen a therapeutic treatment of a Dysbiosis IBD related or of IBD.

The present inventors therefore characterized and compared the intestinal microbiota composition of both pediatric and adult IBD subjects. The present approach allowed to sequence the entire 16S rRNA bacterial gene, and together with the present optimized bioinformatic pipeline, to obtain accurate organism identification down to the species level. By performing differentially abundance statistical analysis, microbial species specific to IBD subjects but also to CD and UC individuals were identified. The present method, and the derived Dysbiosis index - IBD related, allows to distinguish IBD patients from healthy subjects and to differentiate CD patients from UC patients, based on characteristic fecal microbial signature. Our results constitutes a non-invasive fast diagnostic tool and also helps to monitor IBD pathogenesis over time. It is therefore an object of the present invention a method for identifying and optionally quantifying microorganisms in a sample obtained from a subject comprising:

(a) extracting genomic DNA from the sample to obtain an extracted genomic DNA sample ;
(b) amplifying full-length 16S rRNA gene from the extracted genomic DNA sample to get 16S rRNA gene amplicons ;
(c) further amplifying 16S rRNA gene amplicons generated in step (b) to ligate molecular barcodes;
(d) Ligating adapters to the amplified amplicons of step (c) ;
(e) Sequencing adapter-amplicons of step (d) and subsequent generating sequencing reads ;
(f) Data analysing the sequencing reads generated in step (e) to identify, and optionally quantify, microorganisms

wherein the method is characterized in that:

i. in step (b) the amplification is carried out with primers 27f (5' - TTTCTGTTGGTGCTGATATTGCAGRGTTYGATYMTGGCTCAG - 3' (SEQ ID NO: 1)) and 1492r (5' - ACTTGCCTGTCGCTCTATCTTCCGGTTACCTTGTTACGACTT - 3' (SEQ ID NO:2)) and/or
ii. the amplification of step (b) comprises polymerase chain reaction (PCR) and the annealing temperature of the 16S rRNA gene PCR of step (b) is 51°C and/or
iii. the barcodes are ligated using 5'-phospated primers

and wherein the method is capable of allowing species-level classification of the microorganisms identified or quantified in the sample.

In the primers 27f and 1492r, the 22 nucleotides 5' tails were added to the primers to serve as the annealing sites for the barcoding primers while the remaining 20 nucleotides are specific for the 16s gene; Y and M are degenerated bases.

In particular in the context of the present invention, the nucleotides indicated with R, Y, M, H, V or W are indicated with IUPAC nucleotide codes. Therefore, in the herein mentioned primers, R is A or G, Y is C or T, M is A or C, H is A or C or T, V is A or C or G and W is A or T.

Steps (c) and (d) may also be performed in a single step comprising PCR.

The primers 27f and 1492r select and amplify only the full-length 16S rRNA gene of the microorganism(s).

In step (c) the amplification comprises PCR and is preferably carried out with primers which recognize the sequences of the 5' tails of the primers 27f and 1492r, anneal to such sequences and amplify the 16S rRNA gene amplicons generated in step (b) plus the same barcode.

The use of the above mentioned 5'-phosphated primers allows to remove a step of enzymatic phosphorylation and a subsequent purification. Amplicons phosphorylation is a crucial step in Oxford Nanopore library preparation, known as "End-prep step" (Oxford Nanopore Technology), the enzymatic-mediated phosphorylation is required for the subsequent sequencing adapter ligation. The use of 5'-phosphorilated primers allows to skip the "End-prep" step, saving time and reducing the cost of the procedure (as no reaction and clean-up are performed).

The adapters of the above step (d) are preferably adapters for nanopore sequencing. The adapters of the above step (d) are preferably adapters part of the Ligation Sequencing Kit (SQK-LSK112 or SQK-LSK114) manufactured by Oxford Nanopore Technologies.

Preferably, the sample is a biological sample obtained from a subject, preferably the biological sample is a solid and/or liquid biological sample, such as a faecal, stool, swab, preferably nasal, skin or vaginal swab, blood, synovial fluid or respiratory wash sample, or any combination thereof, or wherein the sample is a pharmaceutical preparation.

Preferably, the amplicons of step (b) are ~1.5 Kb in length.

Preferably, the ligation of molecular barcodes of step (c) enables samples multiplexing.

Preferably, the sequencing of step (e) is Nanopore sequencing, preferably Oxford Nanopore sequencing.

Preferably, the further amplification of step (c) is carried out with primers selected from Tables 2 and 3 and/or are listed in Tables 2 and 3.

Preferably such primers are 5'-phospated primers.

Preferably, the microorganism is a pathogen, an opportunistic and/or a commensal microorganism.

Preferably the method identifies and/or quantify a bacterial taxa present with at least 0.01% relative abundance in the bacterial community of the samples.

Preferably the method results in redundant detection of the microorganism at the phylum level, genus level and species level.

Preferably the amplification of step (b) and/or (c) comprises polymerase chain reaction (PCR). Preferably the data analysis enables i) trimming and filtering of low quality reads, ii) detection of putative chimeric reads, iii) profiling bacterial species at ≥ 97% accuracy and iv) generate final report including relative abundances and species of interest.

Another object of the invention is a method for identifying and optionally quantifying the microorganisms present in the gut microbiota or blood, or urine, or synovial fluid, or pulmonary wash sample of the subject or in sample, such as a pharmaceutical preparation, the method comprising:

(a) providing a biological sample obtained from the subject or in the sample; and
(b) identifying microorganism nucleic acids in the biological sample or in the sample according to the method as described herein.

The pharmaceutical preparation is preferably a preparation that needs to be declared microbiological of complaint.

A further object of the invention is a method for providing an optimized therapy for a subject based on the identification of microorganisms present in the gut microbiota of the subject carried out with the method of the present invention, preferably wherein the optimized therapy to the patient is faster than standard of care.

Another object of the invention is a method for providing personalised probiotic intake, wherein the probiotic present in the personalised probiotic intake is not detected by the method of the present invention, and is preferably one or more of the following species: *Bifidobacterium breve, Lactobacillus acidophilus, Lacticaseibacillus casei, Lactobacillus crispatus, Lactobacillus gasseri, Lacticaseibacillus paracasei, Lactiplantibacillus plantarum Limosilactobacillus reuteri, Lacticaseibacillus rhamnosus, Ligilactobacillus salivarius, Bifidobacterium longum, Bifidobacterium infantis, Bifidobacterium animalis, Lactobacillus helveticus , Bifidobacterium bifium , Lactobacillus delbrueckii* subsp. *Bulgaricus , Escherichia coli Nissle, Enterococcus faecium.*

A further object of the invention is a method for the prognosis and/or for determining the severity risk and/or disease development risk of an Inflammatory Bowel Disease (IBD), such as ulcerative colitis (UC) or Crohn's disease (CD), the method comprising the method of the invention, wherein when the method is for the prognosis and/or for determining the severity risk the subject is an IBD patient.

Another object of the invention is a method for the diagnosis and/or prognosis of a Dysbiosis IBD related or of IBD and/or for the monitoring of the efficacy of a therapeutic treatment of a Dysbiosis IBD related or of IBD and/or for the screening of a therapeutic treatment of a Dysbiosis IBD related or of IBD comprising the steps of:

i. measuring a first total abundance of four or more bacterial species which are known to be increased in the IBD in a sample obtained from the subject;
ii. measuring a second total abundance of four or more bacterial species which are known to be decreased in the IBD in said sample;
iii. calculating the log of the ratio of the first total abundance over the second total abundance to obtain a Microbiome Index for Dysbiosis IBD related (MIDIBD)
iv. comparing the obtained MIDIBD with reference values.

The reference values are calculated from healthy patient or from patients who are not affected by the IBD or by Dysbiosis IBD related.

Preferably the species increased are selected from the group consisting of: *Ruminococcusgnavus, Bacteroides dorei, Anaerostipes hadrus, Veillonella parvula, Blautia wexlerae, Eubacterium halii* and/or

the species decreased are selected from butyrate producing-bacterial species, preferably they are selected from the group consisting of: such as *Faecalibacterium prausnitzii, Roseburia intestinalis, Clostridium leptum, Eubacterium rectale, Eubacterium hallii, Clostridium butyricum.* Preferably the abundance of the bacterial species is measured by carrying out the method of the invention.

The present invention will be illustrated by means of non-limiting examples in reference to the following figures.

Figure 1: Shannon and Simpson diversity in the faecal samples.

Figure 2: Clustering of the faecal samples based on their species-level bacterial community structure.

Figure 3: Dissimilarity in bacterial community structure at species- and genus-level taxonomy between technical replicates of the same sample and different faecal samples. The bold lines show the median values, the boxes cover from the first to the third quartiles, and the whiskers extend to the data extremes.

Figure 4: Clustering of the faecal samples based on their genus-level bacterial community structure.

## DETAILED DESCRIPTION OF THE INVENTION

The term "subject" as used herein, generally refers to an animal or other organism, such as a mammalian species (e.g., human), avian (e.g., bird) species, or plant. Mammals include, but are not limited to, murines, simians, humans, farm animals, sport animals, and pets. A subject can be an individual that has or is suspected of having a disease or a pre-disposition to the disease, or an individual that is in need of therapy or suspected of needing therapy. A subject can be a patient. The term "sample" as used herein, generally refers to any sample containing or suspected of containing a nucleic acid molecule. For example, a subject sample can be a biological sample containing one or more nucleic acid molecules. The biological sample can be obtained (e.g., extracted or isolated) from a bodily sample of a subject that can be selected from blood (e.g., whole blood) , plasma, serum, urine, saliva, mucosal excretions, sputum, stool and tears. The bodily sample can be a fluid or tissue sample (e.g., skin sample) of the subject. In some examples, the sample is obtained from a cell-free bodily fluid of the subject, such as whole blood. In such instance, the sample can include cell-free DNA and/or cell-free RNA. In some other examples, the sample is an environmental sample (e.g., soil, waste, ambient air and etc. ), industrial sample (e.g., samples from any industrial processes), and food samples (e.g., dairy products, vegetable products, and meat products).

The term "microbes", "microorganisms" as used herein should be taken broadly. It refers to any single-celled organisms, bacteria, archaea, protozoa, and unicellular fungi and protists. By way of example, the microorganisms may include Proteobacteria (such as Pseudomonas, Enterobacter, Stenotrophomonas, Burkholderia, Rhizobium, Herbaspirillum, Pantoea, Serratia, Rahnella, Azospirillum, Azorhizobium, Azotobacter, Duganella, Delftia, Bradyrhizobiun, Sinorhizobium and Halomonas), Firmicutes (such as Bacillus, Paenibacillus , Lactobacillus, Mycoplasma, and Acetobacterium), Actinobacteria (such as Streptomyces , Rhodococcus, Microbacterium, and Curtobacterium), and the fungi Ascomycota (such as Trichoderma, Ampelomyces, Coniothyrium, Paecoelomyces, Penicillium, Cladosporium, Hypocrea, Beauveria, Metarhizium, Verticullium, Cordyceps, Pichea, and Candida, Basidiomycota (such as Coprinus, Corticium, and Agaricus) and Oomycota (such as Pythium, Mucor, and Mortierella). "Nucleic acid", "oligonucleotide" and "polynucleotide" refer to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. The term nucleic acid is used interchangeably with gene, cDNA, and mRNA encoded by a gene.

The term "sequencing" as used herein refers to sequencing methods for determining the order of the nucleotide bases-adenine, guanine, cytosine, and thymine- in a nucleic acid molecule (e.g., a DNA or RNA nucleic acid molecule).

The term "barcode" as used herein, refers to any unique, non-naturally occurring, nucleic acid sequence that may be used to identify the originating genome of a nucleic acid fragment.

The method of the invention may be also used for detecting, sequencing, identifying, and characterizing nonspecific pathogens in one or more samples.

The method analyses the nucleic acids of the microorganism.

The cells in the samples may be lysed. The sequencing of the microorganism or of the pathogen nucleic acid is performed preferably by nanopore sequencing.

The extraction of DNA preferably comprises magnetic bead DNA extraction, phenol-chloroform extraction, ethanol or isopropanol extraction, minicolumn purification, or any combination thereof.

The adding of adapters may comprise the use of PCR, ligation enzymes, transposase enzymes, or any combination thereof.

The sequence data analysis is preferably performed remotely through a cloud computing platform.

Preferably, the polymerase used in the PCR of present method is the LongAmp *Taq* DNA polymerase (catalog# M0323S/L) manufactured by New England Biolabs Inc. supplied as part of the LongAmp *Taq* 2X Master Mix (catalog# M0287S/L).

Preferably, in case of stool samples the template amount is 0.1 ng DNA and the number of cycles is 25.

Preferably the barcodes are adapted from Oxford Nanopore PCR barcoding expansion kit (EXP-PBC096) to optimize and accelerate library preparation methods. The present protocol is compatible with PCR barcoding expansion kit family (EXP-PBC001, EXP-PBC096). Preferably a 5' phosphate is added to the primers.

Preferably, the further amplified amplicons of step (c) are pooled in libraries subjected to sequencing adapter ligation with the NEBNext Quick Ligation Module (New England Biolabs) before sequencing.

Preferably the sequencing is performed with a Nanopore Ligation Sequencing kit, preferably with Nanopore Ligation Sequencing Kit SQK-LSK114 or with previous Ligation Sequencing kits (SQK-LSK109, -LSK110, and -LSK112).

The inflammatory Bowel Disease (IBD) is preferably ulcerative colitis (UC) or Crohn's disease (CD).

In the personalised probiotic intake the species that will be recommended are the ones labelled as "Undetected" in each person test report (see e.g. below).

| Species | Abundance |
|---|---|
| Bifidobacterium breve | Undetected |
| Lactobacillus acidophilus | Undetected |
| Lacticaseibacillus casei | Undetected |
| Lactobacillus crispatus | Undetected |
| Lactobacillus gasseri | Undetected |
| Lacticaseibacillus paracasei | Undetected |
| Lactiplantibacillus plantarum | Undetected |
| Limosilactobacillus reuteri | Undetected |
| Lacticaseibacillus rhamnosus | Undetected |
| Ligilactobacillus salivarius | Undetected |
| Bifidobacterium longum | Detected |
| Bifidobacterium infantis | Detected |
| Bifidobacterium animalis | Detected |

Preferably the optimized therapy or the personalised probiotic intake comprises the microorganism that are not identified with the method herein disclosed.

## EXAMPLES

## EXAMPLE 1

## MATERIALS AND METHODS

## ILLUMINA AND NANOPORE SEQUENCING COMPARISON

This experiment included the ZymoBIOMICS Gut Microbiome Standard (Zymo Research, Irvin CA, USA), hereafter referred to as the mock community, which is a mixture of 14 bacterial-, 1 archaeal-, and 2 fungal species, and faecal samples collected from six individuals: sample A1 from a 2-year-old male child, samples AN1 and S1X from adult females, samples B1 and MD01 from adult males, and sample S2P from an adult male diagnosed with irritable bowel syndrome (IBS). The faecal material was sampled with Danastool Sample Collection Microbiome Kit (Danagen, Barcelona, Spain). The samples were homogenised by vigorous shaking and transported to the laboratory at room temperature. DNA was extracted on the day of sample collection. The Danastool tubes were vortexed and from each sample, a 200 $\mu$l aliquot was extracted with MagMAX Microbiome Ultra Nucleic Acid Isolation Kit (Applied Biosystems, Waltham MA, USA) using a KingFisher Flex Purification System (Thermo Fisher Scientific, Waltham MA, USA). In case of the mock community, a 75 $\mu$l aliquot was extracted with the same protocol. The DNA concentration in the extracts was determined with Qubit 1X dsDNA High Sensitivity Kit (Invitrogen, Waltham MA, USA).

## Sequencing library preparation:

Each DNA extract was processed in three technical replicates. The 16S rRNA gene was amplified with primers 27f (5' - TTTCTGTTGGTGCTGATATTGCAGRGTTYGATYMTGGCTCAG - 3' (SEQ ID NO:1)) and 1492r (5' - ACTTGCCTGTCGCTCTATCTTCCGGTTACCTTGTTACGACTT - 3' (SEQ ID NO:2)). The 22 nt 5' tails were added to the primers to serve as the annealing sites for the barcoding primers. The PCRs were carried out in 12.5 $\mu$l total volume containing 6.25 $\mu$l LongAmp Taq 2X Master Mix (New England Biolabs, Ipswich MA, USA), the primers in 400 nM concentration, and 0.05 ng template DNA. The reactions were run in a T100 thermal cycler (BioRad, Hercules CA, USA). The initial denaturation at 95 °C for 4 minutes was followed by 30 cycles of 95 °C for 20 seconds, 51 °C for 30 seconds, and 65 °C for 4 minutes. The final extension was 5 minutes at 65 °C. The PCR products were check on a 2% agarose gel and cleaned with 0.8x1 × Agencourt AMPure XP (Beckman Coulter, Indianapolis IN, USA). The purity and yield of the PCR products were assessed with a NanoDrop 8000 spectrophotometer (Thermo Fisher Scientific).

Barcodes and adapters were added in a second PCR using primers from the Nanopore PCR barcoding expansion kit EXP-PBC001 (Oxford Nanopore Technologies, Oxford, UK). The reactions contained in 25 $\mu$l total volume 12.5 $\mu$l LongAmp Taq 2X Master Mix, 0.5 $\mu$l barcoding primer mix, and 25 ng 16S rRNA gene PCR product. The reactions were run in a T100 thermal cycler with initial denaturation at 95 °C for 3 minutes, 12 cycles of 95 °C for 15 seconds, 62 °C for 15 seconds, and 65 °C for 4 minutes, and final extension for 5 minutes at 65 °C. PCR products were check on a 2% agarose gel, cleaned with 1× Agencourt AMPure XP, and quantified with a NanoDrop 8000 spectrophotometer.

Since the EXP-PBC001 kit contains 12 different barcodes, the PCR products had to be split between, two libraries one containing the technical replicates of samples A1, AN1, B1, and the mock community, and the other the technical replicates of samples MD01, S1X, and S2P. The libraries were pooled and subjected to DNA repair and end-prep with the NEBNext Companion Module for ONT Ligation Sequencing (New England Biolabs) in 60 $\mu$l volume containing 1 $\mu$g DNA. The reactions were incubated in a T100 thermal cycler at 20 °C for 5 minutes followed by 65 °C for 5 minutes. The libraries were cleaned with 1× Agencourt AMPure XP and prepared for sequencing with the Nanopore Ligation Sequencing Kit SQK-LSK112. The two libraries were sequenced on separate R9.4.1 flow cells loading 5 fmol DNA. The flow cells were run on a GridION sequencer with real-time super accurate basecalling and a filter threshold of average Q-score 11.

Illumina libraries were prepared at Novogene (Cambridge, UK). The V4 region of the 16S rRNA gene was amplified with primers 515f (5' - GTGCCAGCMGCCGCGGTAA - 3' (SEQ ID NO:3)) and 506r (5' - GGACTACHVGGGTWTCTAAT - 3' (SEQ ID NO:4)) and the PCR products were sequenced on a NovaSeq 6000 generating 250 nt paired-end reads. The reads were demultiplexed and the primer sequences trimmed by Novogene.

**Data Processing:**

Both the Illumina and Nanopore reads were processed with a pipeline widely used in microbiome analysis and the PKSSU4.0 database (Yoon et al. 2017) to remove non-target, low-quality, and chimeric reads, and to perform the taxonomic classification of the filtered sequences. Briefly, the pipeline merges paired reads, deletes reads that are <100 bp, or >2000 bp, or have an average Q-score <25, or are not predicted to be 16S sequences. The dataset is then de-replicated and taxonomic assignment is done with VSEARCH (Rognes et al. 2016) against the pipeline widely used in microbiome analysis 16S database. Reads that do not match any reference sequence are subjected to chimera detection with UCHIME (Edgar et al. 2011) using a manually curated chimera-free reference database. Sequences that don't have a match in the chosen 16S database with at least 97% similarity are clustered into 97% OTUs with UCLUST (Edgar 2010). Singletons are ignored in the OTU binning. Up to 100,000 reads were uploaded to the pipeline from each sample. Since the pre-processing steps of the pipeline chosen, widely used in microbiome analysis, were not designed for Nanopore data, it was necessary to trim and filter the Nanopore reads beforehand. Trimming was done with cutadapt 3.5 (Martin 2011) to remove adapter, barcode, and primer sequences. The presence of at least 15 bases of each primer sequence was required with no more than 0.2 error. Reads that did not have recognisable primer sequences on both ends were discarded. The filterAndTrim function of the dada2 1.18.0 R package (Callahan et al. 2016) was used to discard reads that were shorter than 1300 bp or longer than 1600 bp, had ambiguous bases, or more than 25 expected errors. The results of the chosen available pipeline were organised into three sets: one containing the Nanopore data, one the Illumina data, and one merging the data from both sequencing platforms. Hereafter these are referred to as the Nanopore set, the Illumina set, and the merged set respectively. The 16S reference database has different versions to match various regions of 16S sequences. In these, taxa that are indistinguishable by the given 16S region are merged into groups. Since the Nanopore reads covered all the variable regions of the 16S rRNA gene while the Illumina reads covered only one, these groups were different for the two datasets. If the Nanopore and Illumina results were directly merged into a single data matrix, several taxa present in the Nanopore data would be found missing from the Illumina data not because Illumina sequencing failed to detect their 16S rRNA gene sequences but because the short reads couldn't distinguish them from the sequences of related taxa. To resolve this issue, the merged set was created with a version of the 16S reference database that joins species that either the V4 or V1 - V9 regions can't distinguish into groups. Consequently, the classification results from the Nanopore data were not identical in the Nanopore set and the merged set. In the analysis of the results, the merged set was used only when it was inevitable to have the Nanopore and Illumina data in a single data matrix.

**Statistical analysis**

The merged set was used only in the cluster analysis and the permutational multivariate analysis of variance (PERMANOVA). All other results were obtained from the Nanopore and Illumina sets. The analyses were carried out in R 4.2.1 (https://www.r-project.org) with functions from the vegan package 2.6-2 (Oksanen et al. 2022). Cluster analysis was performed with the hclust function. The data matrix was relativised before calculating Bray-Curtis dissimilarities. Clusters were found with the unweighted pair group method with arithmetic mean (UPGMA). PERMANOVA based on Bray-Curtis dissimilarities was used with the adonis function to test the marginal effects of sequencing method and the differences between faecal samples. To quantify the proportion of variance explained by these factors, $\omega^2$ values were calculated from the PERMANOVA results with the adonis_OmegaSq function of the MicEco package 0.9.17 (Russel 2021). To compare the differences in bacterial community structure between faecal samples to the differences between technical replicates of the same sample, Bray-Curtis dissimilarities were calculated from the Nanopore and Illumina sets and compared with Welch two sample t-tests. Shannon and Simpson diversity indices were calculated with the diversity function of the vegan package. Rarefaction curves were generated with the rarecurve function.

**RESULTS**

**Sequencing yield**

Nanopore sequencing generated 4,289,400 reads in total passing the quality threshold in MinKNOW (Table 4) with median quality score 17.6 and 17.5 in the two libraries and a median read length of 1637 nt in both libraries. 90.7% of the reads passed trimming and 55.7% (2387309 in total) the quality filter corresponding to 113681 $\pm$ 16289 reads per sample. Illumina sequencing yielded 2509425 read pairs in total or 119496 $\pm$ 11038 reads per sample. The read length was 253 nt and 93% of the bases had quality scores > 30. From each sample, up to 100000 Nanopore reads or Illumina read pairs were processed with the chosen pipeline . 1.7% of the Nanopore reads were found to be non-16S, in total 33 reads to be non-bacterial, and 19.8% to be chimeric (Table 5). 77438 $\pm$ 5496 reads per sample passed the pipeline, 98.7% of which could be classified into species, phylotypes, or species groups. In comparison, 0.7% of the Illumina reads were low-quality or non-16S, 0.4% non-bacterial, and 8.2% chimeric (Table 6). 90817 $\pm$ 1481 reads per sample passed the pipeline, 94.5% of which could be classified into species, phylotypes, or species groups.

**Table 4: Sequencing yield**

| Sample | Illumina raw read pairs | Nanopore raw reads | Nanopore trimmed reads | Nanopore quality filtered reads |
|---|---|---|---|---|
| **Mock** | 104889 | 178513 | 161733 | 100506 |
| **Mock** | 126117 | 181006 | 164518 | 101523 |
| **Mock** | 136677 | 162365 | 147664 | 91350 |
| **A1** | 126280 | 199489 | 180961 | 111640 |
| **A1** | 138564 | 198499 | 179941 | 111761 |
| **A1** | 108634 | 187606 | 169693 | 105160 |
| **B1** | 110781 | 161598 | 146228 | 90066 |
| **B1** | 130274 | 177145 | 159998 | 99062 |
| **B1** | 118184 | 165345 | 149378 | 92191 |
| **AN1** | 120367 | 190866 | 173056 | 106591 |
| **AN1** | 125680 | 181985 | 164611 | 100666 |
| **AN1** | 112429 | 182352 | 164744 | 101844 |
| **MD01** | 119083 | 250784 | 227059 | 139562 |
| **MD01** | 109324 | 230994 | 209913 | 127798 |
| **MD01** | 106072 | 224344 | 204196 | 125022 |
| **S1X** | 113130 | 217891 | 197947 | 121585 |
| **S1X** | 136979 | 236344 | 214118 | 131186 |
| **S1X** | 130658 | 240542 | 218454 | 133814 |
| **S2P** | 108602 | 245526 | 223887 | 127347 |
| **S2P** | 121263 | 236612 | 214531 | 133116 |
| **S2P** | 105438 | 239594 | 217607 | 135519 |

**Table 5: The number of Nanopore reads at each stage of the chosen reference pipeline**

| Sample | Uploaded | Low quality or not 16S | Not bacterial | Chimeric | Passed | Classified to species, phylotypes, or species groups |
|---|---|---|---|---|---|---|
| Mock | 100000 | 1273 | 0 | 13431 | 85296 | 84551 |
| Mock | 100001 | 1293 | 0 | 14903 | 83805 | 83085 |
| Mock | 91350 | 1119 | 0 | 12583 | 77648 | 76985 |
| A1 | 100000 | 1615 | 0 | 23154 | 75231 | 74834 |
| A1 | 100000 | 1493 | 0 | 22369 | 76138 | 75776 |
| A1 | 100000 | 1554 | 0 | 23054 | 75392 | 75027 |
| B1 | 90066 | 1799 | 0 | 22104 | 66163 | 65172 |
| B1 | 99062 | 2023 | 0 | 25646 | 71393 | 70284 |
| B1 | 92191 | 1786 | 0 | 22563 | 67842 | 66779 |
| AN1 | 100000 | 1691 | 0 | 20953 | 77356 | 76633 |
| AN1 | 100000 | 1717 | 0 | 20406 | 77877 | 77160 |
| AN1 | 100000 | 1684 | 10 | 20451 | 77855 | 77082 |
| MD01 | 100000 | 1769 | 5 | 15234 | 82992 | 82217 |
| MD01 | 100000 | 1952 | 10 | 18327 | 79711 | 79027 |
| MD01 | 100000 | 1883 | 2 | 16308 | 81807 | 81082 |
| S1X | 100000 | 2205 | 0 | 25069 | 72726 | 69749 |
| S1X | 100000 | 2176 | 6 | 24719 | 73099 | 70287 |
| S1X | 100000 | 2185 | 0 | 24810 | 73005 | 70036 |
| S2P | 100000 | 1377 | 0 | 14416 | 84207 | 83562 |
| S2P | 100000 | 1473 | 0 | 16577 | 81950 | 81130 |
| S2P | 100000 | 1308 | 0 | 13977 | 84715 | 83943 |

**Table 6: The number of Illumina reads at each stage of the chosen reference pipeline**

| Sample | Uploaded | Low quality or not 16S | Not bacterial | Chimeric | Passed | Classified to species, phylotypes, or species groups |
|---|---|---|---|---|---|---|
| Mock | 100000 | 825 | 119 | 8007 | 91049 | 87586 |
| Mock | 100000 | 934 | 165 | 6860 | 92041 | 87598 |
| Mock | 100000 | 1094 | 253 | 6999 | 91654 | 86840 |
| A1 | 100000 | 527 | 18 | 7136 | 92319 | 89941 |
| A1 | 100000 | 486 | 21 | 6550 | 92943 | 90640 |
| A1 | 100000 | 507 | 22 | 6943 | 92528 | 90383 |
| B1 | 100000 | 526 | 91 | 7088 | 92295 | 87673 |
| B1 | 100000 | 686 | 17 | 7636 | 91661 | 86507 |
| B1 | 100000 | 505 | 43 | 7143 | 92309 | 88140 |
| AN1 | 100000 | 737 | 55 | 8143 | 91065 | 86083 |

(continued)

| Sample | Uploaded | Low quality or not 16S | Not bacterial | Chimeric | Passed | Classified to species, phylotypes, or species groups |
|---|---|---|---|---|---|---|
| AN1 | 100000 | 506 | 44 | 8150 | 91300 | 86315 |
| AN1 | 100000 | 690 | 57 | 8189 | 91064 | 85718 |
| MD01 | 100000 | 537 | 2448 | 8964 | 88051 | 82813 |
| MD01 | 100000 | 545 | 2054 | 8823 | 88578 | 83297 |
| MD01 | 100000 | 669 | 1895 | 9134 | 88302 | 83785 |
| S1X | 100000 | 702 | 180 | 8651 | 90467 | 85917 |
| S1X | 100000 | 659 | 136 | 8431 | 90774 | 85948 |
| S1X | 100000 | 671 | 301 | 8448 | 90580 | 86298 |
| S2P | 100000 | 809 | 108 | 10299 | 88784 | 79804 |
| S2P | 100000 | 855 | 51 | 8848 | 90246 | 80417 |
| S2P | 100000 | 787 | 93 | 9977 | 89143 | 80501 |

**Mock community**

The mock community contained 18 bacterial strains representing 14 species. With Nanopore sequencing, 35, 38, and 39 species-level taxa (species with valid names, phylotypes, and species groups) were identified in the three technical replicates of the mock community. In contrast, 2076, 2268, and 2548 species-level taxa were found in the Illumina data. Due to this gross overestimation of richness, we tried several approaches to remove noise from the Illumina data. Keeping only taxa that were detected in all three technical replicates left 1073 species-level taxa in the dataset while discarding 4.2%, 5.0%, and 5.2% of the reads of the replicates. Removing species-level taxa that didn't have at least 60 reads in total across the three replicates left 195, 195, and 204 species-level taxa but discarded 9.6%, 10.6%, and 12.4% of the reads. Deleting taxa that didn't reach at least 0.1% relative abundance in any of the replicates left 87, 88, and 92 species-level taxa but removed 14.7%, 15.3%, and 17.8% of the reads.

*Clostridium perfringens* and *Enterococcus faecalis* were detected neither with Nanopore nor with Illumina sequencing and the Illumina data also missed *Salmonella enterica* (Table 7). This is explained by the very low abundance of these species in the mock community. In the Nanopore data, *Faecalibacterium prausnitzii* was represented by only a few reads but many reads were classified into two *Faecalibacterium* phylotypes: *Faecalibacterium* GG697149 and *Faecalibacterium* NMTZ. *Faecalibacterium* GG697149 was also abundant in the Illumina data, as well as *Roseburia cecicola* and *Veillonella dispar.* These are very likely results of misclassification of *Faecalibacterium prausnitzii, Roseburia hominis,* and *Veillonella rogosae* reads into species with very similar 16S rRNA sequences. When these misclassified reads were included, 96.2%, 96.3%, and 96.4% of the Nanopore reads from the three replicates were from species that were part of the mock community. Furthermore, nearly 100% of the reads were classified into genera represented in the mock community. In contrast, only 64.7%, 68.4%, and 72.5% of the Illumina reads were classified into the species of the mock community. This improved somewhat when the results were classified at higher taxonomic levels: 69.8%, 72.7%, and 76.3% of the Illumina reads of the three replicates were from genera and 77.8%, 81.0%, and 82.6% from families that were present in the mock community. Removing species that didn't reach at least 0.1% relative abundance in any of the replicates increased the proportion of Illumina reads classified into the species of the mock community to 75.9%, 83.2%, and 85.6%.

Apart from the misclassification of the *Faecalibacterium prausnitzii* reads, the Nanopore results were close to the composition of the mock community (Table 7). The largest deviations were in the cases of *Akkermansia muciniphila* and *Clostridioides difficile,* the relative abundances of which were overestimated. The differences between the three replicates were minimal. In contrast, the Illumina results showed large variation between the replicates especially in the cases of *Bacteroides fragilis, Fusobacterium nucleatum,* and *Prevotella corporis.* These differences were also present in the genus-level classification results (Table 8). The QIIME2 and mothur pipelines gave similar results (Table 9).

**Table 7: Relative abundance (%) of the bacterial species in the mock community compared to the Nanopore and Illumina results**

| Species | Composition | Nanopore | | | Illumina unfiltered | | | Illumina filtered | | |
|---|---|---|---|---|---|---|---|---|---|---|
| *Akkermansia muciniphila* | 0.97 | 1.61 | 1.68 | 1.58 | 0.67 | 0.58 | 0.39 | 0.79 | 0.69 | 0.47 |
| *Bacteroides fragilis* | 9.94 | 13.97 | 14.21 | 13.88 | 7.76 | 6.85 | 12.60 | 9.10 | 8.09 | 15.33 |
| *Bifidobacterium adolescentis* | 8.78 | 6.66 | 7.20 | 6.92 | 8.11 | 9.54 | 8.17 | 9.51 | 11.27 | 9.93 |
| *Clostridioides difficile* | 2.62 | 5.40 | 5.05 | 5.41 | 1.69 | 2.70 | 2.15 | 1.98 | 3.19 | 2.61 |
| *Clostridium perfringens* | 0.0002 | 0 | 0 | 0 | 0 | 0 | 0 | 0 | 0 | 0 |
| *Enterococcus faecalis* | 0.0009 | 0 | 0 | 0 | 0 | 0 | 0 | 0 | 0 | 0 |
| *Escherichia coli group* | 12.12 | 8.72 | 8.79 | 8.51 | 5.41 | 8.42 | 7.53 | 6.34 | 9.94 | 9.16 |
| *Faecalibacterium prausnitzii* | 17.63 | 0.0035 | 0.0048 | 0.0039 | 11.56 | 15.94 | 15.69 | 13.55 | 18.83 | 19.08 |
| *Fusobacterium nucleatum* | 7.49 | 7.57 | 7.33 | 7.03 | 9.08 | 13.60 | 4.47 | 10.65 | 16.06 | 5.44 |
| *Lactobacillus fermentum* | 9.63 | 5.67 | 5.65 | 6.00 | 2.30 | 3.11 | 2.25 | 2.69 | 3.68 | 2.73 |
| *Prevotella corporis* | 4.98 | 6.57 | 6.42 | 6.47 | 5.55 | 0.94 | 4.14 | 6.51 | 1.11 | 5.03 |
| *Roseburia hominis* | 9.89 | 7.10 | 7.07 | 7.06 | 4.08 | 5.49 | 4.79 | 4.79 | 6.48 | 5.83 |
| *Salmonella enterica* | 0.009 | 0.018 | 0.024 | 0.012 | 0 | 0 | 0 | 0 | 0 | 0 |
| *Veillonella rogosae* | 15.87 | 18.44 | 18.69 | 19.36 | 2.69 | 1.71 | 1.94 | 3.16 | 2.02 | 2.35 |
| **Total** | **99.9** | **81.7** | **82.1** | **82.2** | **58.9** | **68.9** | **64.1** | **69.1** | **81.4** | **78.0** |
| *Faecalibacterium GG697149* | | 4.39 | 4.54 | 4.42 | 1.44 | 0.93 | 1.15 | 1.68 | 1.09 | 1.40 |
| *Faecalibacterium NMTZ* | | 9.62 | 9.15 | 9.31 | 0 | 0 | 0 | 0 | 0 | 0 |
| *Roseburia cecicola* | | 0.043 | 0.043 | 0.046 | 1.43 | 1.1 | 1.12 | 1.68 | 1.29 | 1.36 |
| *Veillonella dispar* | | 0.45 | 0.43 | 0.39 | 2.92 | 1.58 | 2.02 | 3.43 | 1.87 | 2.46 |
| **Total** | **99.9** | **96.2** | **96.3** | **96.4** | **64.7** | **72.5** | **68.4** | **75.9** | **85.6** | **83.2** |

The Illumina filtered results were obtained by removing taxa that didn't reach at least 0.1% relative abundance in any of the replicates.

**Table 8: Relative abundance (%) of the bacterial genera in the mock community compared to the Nanopore and Illumina results from the chosen refeerence pipeline**

| Genus | Composition | Nanopore | | | Illumina | | |
|---|---|---|---|---|---|---|---|
| *Akkermansia* | 0.97 | 1.61 | 1.69 | 1.58 | 0.67 | 0.58 | 0.39 |
| *Bacteroides* | 9.94 | 14.06 | 14.27 | 13.95 | 11.26 | 8.73 | 14.96 |
| *Bifidobacterium* | 8.78 | 6.75 | 7.28 | 6.99 | 8.24 | 9.66 | 8.28 |
| *Clostridioides* | 2.62 | 5.43 | 5.06 | 5.43 | 1.69 | 2.70 | 2.15 |
| *Clostridium* | 0.0002 | 0 | 0 | 0 | 0.270 | 0.013 | 0.023 |
| *Enterococcus* | 0.0009 | 0 | 0 | 0 | 0.0868 | 0.0054 | 0.0011 |
| *Escherichia* | 12.12 | 10.82 | 10.97 | 10.63 | 5.41 | 8.42 | 7.53 |
| *Faecalibacterium* | 17.63 | 15.00 | 14.58 | 14.64 | 13.70 | 17.37 | 17.37 |
| *Fusobacterium* | 7.49 | 7.64 | 7.38 | 7.09 | 9.09 | 13.61 | 4.47 |
| *Lactobacillus* | 9.63 | 5.68 | 5.67 | 6.01 | 2.34 | 4.13 | 3.26 |
| *Prevotella* | 4.98 | 6.58 | 6.44 | 6.48 | 5.62 | 0.94 | 4.14 |
| *Roseburia* | 9.89 | 7.15 | 7.11 | 7.12 | 5.79 | 6.78 | 6.11 |
| *Salmonella* | 0.009 | 0.018 | 0.024 | 0.012 | 0 | 0 | 0 |
| *Veillonella* | 15.87 | 19.24 | 19.52 | 20.06 | 5.67 | 3.33 | 3.99 |
| Total | 99.93 | 99.97 | 99.98 | 99.99 | 69.83 | 76.27 | 72.67 |

**Table 9: Relative abundance (%) of the bacterial genera in the mock community compared to the Illumina results from the QIIME2 and mothur pipelines**

| Genus | Composition | Illumina QIIME2 | | | Illumina mothur | | |
|---|---|---|---|---|---|---|---|
| *Akkermansia* | 0.97 | 0.71 | 0.63 | 0.38 | 0.68 | 0.58 | 0.36 |
| *Bacteroides* | 9.94 | 11.30 | 8.93 | 15.36 | 9.51 | 7.85 | 13.95 |
| *Bifidobacterium* | 8.78 | 8.22 | 9.66 | 8.31 | 8.23 | 9.57 | 8.13 |
| *Clostridioides* | 2.62 | 1.75 | 2.75 | 2.19 | 1.69 | 2.60 | 2.10 |
| *Clostridium* | 0.0002 | 0.24 | 0.0060 | 0.015 | 0.23 | 0.012 | 0.019 |
| *Enterococcus* | 0.0009 | 0.11 | 0 | 0 | 0.081 | 0.0037 | 0.00086 |
| *Escherichia* | 12.12 | 5.56 | 8.62 | 7.74 | 5.66 | 8.43 | 7.58 |
| *Faecalibacterium* | 17.63 | 13.73 | 17.86 | 17.58 | 14.20 | 17.74 | 17.59 |
| *Fusobacterium* | 7.49 | 9.13 | 13.80 | 4.47 | 9.22 | 13.60 | 4.39 |
| *Lactobacillus* | 9.63 | 2.43 | 4.25 | 3.43 | 2.28 | 3.10 | 2.25 |
| *Prevotella* | 4.98 | 5.67 | 0.95 | 4.27 | 5.66 | 0.91 | 4.13 |
| *Roseburia* | 9.89 | 4.44 | 5.68 | 5.01 | 4.38 | 5.58 | 4.91 |
| *Salmonella* | 0.009 | 0 | 0 | 0 | 0 | 0 | 0 |
| *Veillonella* | 15.87 | 5.64 | 3.34 | 4.16 | 5.68 | 3.28 | 4.03 |
| Total | 199.93 | 69.0 | 176.5 | 172.9 | 167.6 | 73.3 | 169.5 |

**Faecal samples**

With both sequencing methods, nearly 100% of the reads from the faecal samples could be classified to genera or genus-level phylotypes. At species-level classification, however, 82.1 - 97.4% of the Nanopore reads but only 30.8 - 53.8% of the Illumina reads could be assigned to species with valid names or phylotypes (Table 10). The remaining reads were classified into species groups or could be classified only at a higher taxonomic level.

**Table 10: The percentage of reads classified into species and genera in the faecal samples**

| | Reads classified as species or phylotypes (%) | | Reads classified as genera or phylotype genera (%) | |
|---|---|---|---|---|
| Sample | Nanopore | Illumina | Nanopore | Illumina |
| A1 | 86.2 | 53.0 | 99.98 | 99.40 |
| A1 | 86.2 | 52.8 | 99.99 | 99.44 |
| A1 | 85.6 | 51.5 | 99.98 | 99.51 |
| AN1 | 92.4 | 28.4 | 99.97 | 99.10 |
| AN1 | 92.4 | 30.8 | 99.93 | 99.16 |
| AN1 | 92.0 | 31.1 | 99.98 | 99.27 |
| B1 | 94.8 | 40.2 | 99.96 | 99.35 |
| B1 | 94.5 | 39.7 | 99.97 | 99.02 |
| B1 | 94.7 | 38.7 | 99.95 | 99.33 |
| MD01 | 83.2 | 53.8 | 99.77 | 98.46 |
| MD01 | 82.2 | 53.0 | 99.89 | 99.18 |
| MD01 | 82.1 | 53.5 | 99.83 | 99.06 |
| S1X | 91.8 | 53.8 | 99.79 | 99.27 |
| S1X | 92.0 | 53.1 | 99.90 | 99.25 |
| S1X | 91.9 | 53.6 | 99.75 | 99.34 |
| S2P | 97.4 | 52.3 | 100.00 | 99.24 |
| S2P | 96.8 | 52.5 | 99.98 | 98.95 |
| S2P | 97.3 | 51.1 | 100.00 | 99.12 |

The Illumina data indicated very high bacterial richness in the faecal samples. It identified 187 - 292 species, 258 - 411 species groups, and 791 - 1236 phylotypes in the samples (Table 11). In comparison, 82 - 127 species, 9 - 32 species groups, and 46 - 249 phylotypes were found with Nanopore. Similarly, the samples had 353 - 483 genera according to the Illumina data but only 63 - 94 based on Nanopore (Table 12). However, technical replicates prepared from the same faecal sample were similar to each other in their number of species, species groups, phylotypes, and genera in both the Nanopore and Illumina data.

**Table 11: The number of species-level taxa detected in the faecal samples**

| | Species | | Species groups | | Phylotypes | | Phylotype groups | |
|---|---|---|---|---|---|---|---|---|
| Sample | Nanopore | Illumina | Nanopore | Illumina | Nanopore | Illumina | Nanopore | Illumina |
| A1 | 82 | 214 | 18 | 309 | 46 | 933 | 1 | 115 |
| A1 | 82 | 198 | 15 | 286 | 51 | 791 | 1 | 101 |
| A1 | 88 | 193 | 18 | 315 | 49 | 916 | 1 | 107 |
| AN1 | 127 | 241 | 32 | 352 | 130 | 967 | 1 | 105 |
| AN1 | 117 | 187 | 31 | 298 | 123 | 816 | 1 | 92 |
| AN1 | 122 | 229 | 30 | 316 | 122 | 965 | 1 | 116 |
| B1 | 99 | 213 | 15 | 297 | 164 | 900 | 1 | 102 |
| B1 | 96 | 245 | 14 | 343 | 158 | 1065 | 2 | 117 |

(continued)

| | Species | | Species groups | | Phylotypes | | Phylotype groups | |
|---|---|---|---|---|---|---|---|---|
| Sample | Nanopore | Illumina | Nanopore | Illumina | Nanopore | Illumina | Nanopore | Illumina |
| B1 | 93 | 236 | 11 | 355 | 158 | 864 | 1 | 92 |
| MD01 | 95 | 197 | 25 | 258 | 179 | 801 | 1 | 88 |
| MD01 | 101 | 195 | 25 | 293 | 189 | 946 | 1 | 109 |
| MD01 | 102 | 218 | 24 | 307 | 192 | 1139 | 2 | 123 |
| S1X | 99 | 240 | 18 | 316 | 234 | 1158 | 1 | 129 |
| S1X | 104 | 231 | 20 | 324 | 249 | 1089 | 1 | 110 |
| S1X | 99 | 219 | 20 | 328 | 241 | 1113 | 2 | 122 |
| S2P | 92 | 242 | 16 | 351 | 104 | 1124 | 1 | 135 |
| S2P | 87 | 292 | 11 | 394 | 102 | 1236 | 1 | 128 |
| S2P | 84 | 271 | 9 | 411 | 103 | 1146 | 1 | 120 |

**Table 12: The number of genera detected in the faecal samples**

| | Genera | | Phylotype genera | |
|---|---|---|---|---|
| Sample | Nanopore | Illumina | Nanopore | Illumina |
| A1 | 63 | 392 | 5 | 354 |
| A1 | 64 | 376 | 6 | 318 |
| A1 | 68 | 388 | 6 | 378 |
| AN1 | 94 | 452 | 31 | 418 |
| AN1 | 91 | 360 | 28 | 337 |
| AN1 | 89 | 398 | 29 | 391 |
| B1 | 83 | 395 | 40 | 363 |
| B1 | 76 | 439 | 45 | 442 |
| B1 | 77 | 431 | 42 | 374 |
| MD01 | 89 | 353 | 56 | 315 |
| MD01 | 92 | 392 | 59 | 349 |
| MD01 | 90 | 406 | 65 | 435 |
| S1X | 91 | 415 | 65 | 428 |
| S1X | 94 | 411 | 76 | 410 |
| S1X | 93 | 409 | 72 | 439 |
| S2P | 77 | 435 | 26 | 424 |
| S2P | 72 | 477 | 27 | 494 |
| S2P | 71 | 483 | 26 | 469 |

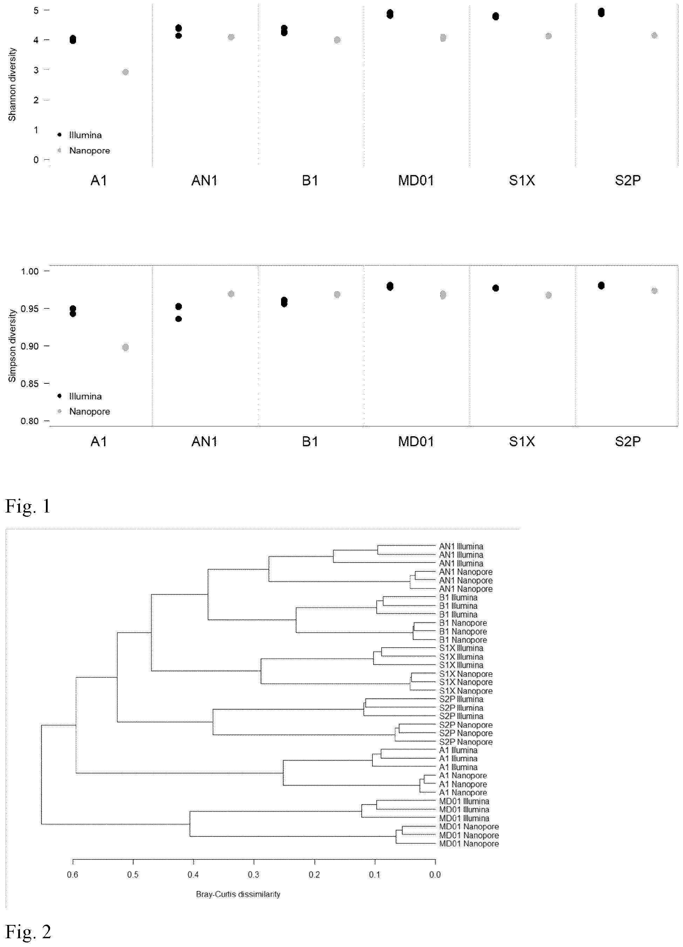

Despite the large difference in the number of species-level taxa detected with Nanopore and Illumina sequencing, the difference in Shannon and Simpson diversity was relatively small. In fact, Simpson diversity was higher according to the Nanopore data than based on the Illumina results in samples AN1 and B1 (Figure 1).

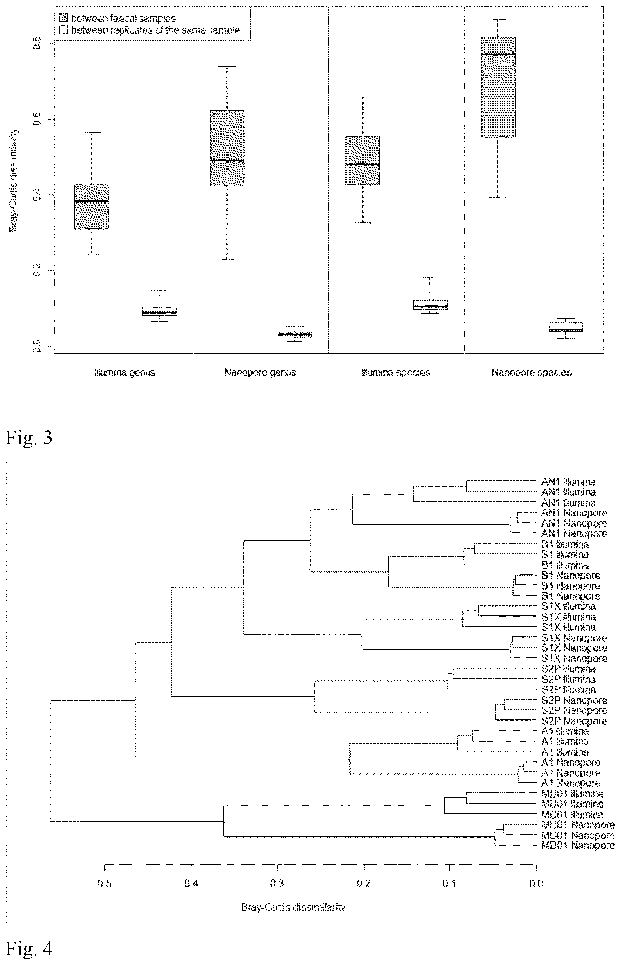

The six stool samples were clearly distinguished based on their bacterial community structures in both the Illumina and Nanopore data (Figure 2). The technical replicates prepared from the same faecal sample with the same sequencing method were very similar. In the Nanopore data, the replicates clustered together at shorter distances indicating less replicate-to-replicate variation than in the Illumina data. In case of every faecal sample, the technical replicates divided into two clusters according to the sequencing method. This shows that the sequencing method clearly affected the bacterial community structure results but this effect was smaller than the differences between the six faecal samples. In fact, the difference between the Nanopore and Illumina results was significant ($p < 0.001$) and accounted for 10.9% of the variation in the bacterial community structure at species-level resolution and 10.0% at genus-level resolution. However, the differences between the six faecal samples explained 81.3% and 82.4% of the variation at species- and genus-level respectively. The clustering results obtained from species- and genus-level taxonomy were very similar (Figure 4).

Differences between the Nanopore and Illumina results were present even at the highest taxonomic level. Illumina identified 46 phyla in the dataset while Nanopore detected only 10. Looking at the most abundant phyla, the relative abundance of *Firmicutes,* and consequentially the *Firmicutes/Bacteroidetes* and *Firmicutes/Actinobacteria* ratios were higher with Nanopore than with Illumina in every faecal sample (Table 13). On the other hand, the relative abundance of *Proteobacteria* was consistently measured to be lower with Nanopore. In case of the other abundant phyla, however, some samples had very similar results in the Illumina and Nanopore datasets (e.g. *Actinobacteria* in sample A1 or *Bacteroidetes* in sample S2P), while others were very different (e.g. *Actinobacteria* in sample S2P or *Bacteroidetes* in sample MD01). The effect of the sequencing method on the results depended on the faecal sample. With the small number of samples included in this study, this interaction impeded the characterisation of the differences in the taxonomic composition results between the Nanopore and Illumina data. Nevertheless, it is clear from the data that even at phylum-level classification, the differences between the Nanopore and Illumina results cannot be removed by applying simple conversion factors.

**Table 13: Relative abundance (% ± SD) of the dominant phyla and their ratios in the faecal samples**

| Sample | Sequencing | *Firmicutes* | *Bacteroidetes* | *Actinobacteria* | *Proteobacteria* | *Verrucomicrobia* | *Firmicutes/Bacteroidetes* | *Firmicutes/Actinobacteria* |
|---|---|---|---|---|---|---|---|---|
| A1 | Nanopore | :79.1 ± 0.3 | 7.6 ± 0.1 | 12.5 ± 0.4 | 0.5 ± 0.01 | 0.25 ± 0.04 | 10.3 ± 0.15 | 6.4 ± 0.23 |
|  | Illumina | 71.6 ± 1.3 | 9.5 ± 0.3 | 12.2 ± 1.0 | 4.6 ± 0.2 | 0.20 ± 0.03 | 7.5 ± 0.36 | 5.9 ± 0.57 |
| AN1 | Nanopore | 64.9 ± 0.2 | 14.2 ± 0.3 | 18.8 ± 0.2 | 2.2 ± 0.2 | 0.0004 ± 0.0008 | 4.6 ± 0.06 | 3.5 ± 0.06 |
|  | Illumina | 50.2 ± 1.2 | 19.0 ± 1.6 | 23.5 ± 1.8 | 4.3 ± 0.2 | 0.39 ± 0.05 | 2.6 ± 0.25 | 2.2 ± 0.23 |
| B1 | Nanopore | 73.1 ± 0.4 | 10.6 ± 0.2 | 15.5 ± 0.4 | 0.8 ± 0.1 | ! 0.012 ± \| 0.002 | 6.9 ± 0.17 | 4.7 ± 0.10 |
|  | Illumina | 62.4 ± 0.6 | 11.4 ± 0.8 | 18.6 ± 0.8 | 5.8 ± 0.4 | 0.22 ± 0.03 | 5.5 ± 0.46 | 3.4 ± 0.12 |
| MD01 | Nanopore | 77.8 ± 0.8 | 1.7 ± 0.1 | 11.3 ± 0.6 | 3.3 ± 0.1 | 5.05 ± 0.17 | 46.7 ± 2.05 | 6.9 ± 0.46 |
|  | Illumina | 60.9 ± 2.4 | 12.7 ± 0.3 | 14.8 ± 1.1 | 4.2 ± 0.5 | 4.55 ± 1.68 | 4.8 ± 0.26 | 4.1 ± 0.51 |
| S1X | Nanopore | 66.9 ± 0.8 | 23.1 ± 0.6 | 8.7 ± 0.3 | 0.7 0.02 | 0.095 ± 0.02 | 2.9 ± 0.10 | 7.7 ± 0.31 |
|  | Illumina | 58.3 ± 1.1 | 21.6 ± 0.8 | 11.0 ± 0.4 | 6.2 ± 0.2 | 0.42 ± 0.02 | 2.7 ± 0.15 | 5.3 ± 0.26 |
| ! S2P | Nanopore | 66.9 ± 1.0 | 28.5 ± 0.6 | 1.0 ± 0.2 | 3.7 ± 0.3 | 0.0012 ± 0.0012 | 2.4 ± 0.06 | 71.5 ± 12.23 |
|  | Illumina | 53.8 ± 1.2 | 26.8 ± 2.0 | 6.7 ± 0.6 | 9.9 ± 0.7 | 0.55 ± 0.07 | 2.0 ± 0.20 | 8.1 ± 0.56 |

To assess the replicability of the results from the two sequencing methods, inventors compared the differences in bacterial community structure among technical replicates prepared from the same faecal sample to the differences between the six faecal samples. Bray-Curtis dissimilarities calculated from the Nanopore and Illumina sets showed that with both sequencing methods, the differences in bacterial community structure between technical replicates were minor compared to the differences between the faecal samples (Figure 2). This was true with both species- and genus-level taxonomy. The difference between the variation among technical replicates of the same sample and the variation among faecal samples was larger in the Nanopore data. Furthermore, the Bray-Curtis dissimilarities between the replicates were significantly smaller with Nanopore than with Illumina (Welch t-test $p < 0.0001$ with both species- and genus-level taxonomy). Thus, the replicability was good with both sequencing methods but better with Nanopore.

## DISCUSSION

The most important concern about Nanopore sequencing is its higher error rate which could undermine the accuracy of the taxonomic classification of the reads. Therefore, our aim was to compare the Nanopore results with the Illumina setup that delivers the highest possible sequencing quality. Hence, we selected the NovaSeq 6000 system and targeted the V4 region of the 16S rRNA gene with which the $2 \times 250$ bp read pairs obtain a near complete overlap. This means that almost all base pairs were sequenced twice. In consequence, hardly any reads had to be removed due to insufficient quality during the processing of the Illumina data. The kit 12 chemistry brought a substantial improvement to the sequence quality of Nanopore but we still lost a very large portion, 44.3%, of the Nanopore reads at the quality filtering step of the data processing. With the recently introduced kit 14 chemistry and R10.4.1 flow cells, it is likely that future studies won't have to expect losing this much data due to insufficient quality. The retained reads, however, had over 98.08% accuracy as estimated from the Q-scores. Since this accuracy is higher than the commonly used 97% similarity threshold to distinguish species, it was sensible to attempt the species-level classification of the reads.

## EXAMPLE 2

### Sample collection

The faecal material was sampled with Danastool Sample Collection Microbiome Kit (Danagen, Barcelona, Spain). The samples were homogenised by vigorous shaking and transported to the laboratory at room temperature. The Danastool tubes were vortexed and from each sample, a 200 $\mu$l aliquot was extracted with MagMAX Microbiome Ultra Nucleic Acid Isolation Kit (Applied Biosystems, Waltham MA, USA) using a KingFisher Flex Purification System (Thermo Fisher Scientific, Waltham MA, USA). In case of the mock community, a 75 $\mu$l aliquot was extracted with the same protocol. The DNA concentration in the extracts was determined with Qubit 1X dsDNA High Sensitivity Kit (Invitrogen, Waltham MA, USA).

### Nanopore sequencing

Each DNA extract was processed in three technical replicates. The 16S rRNA gene was amplified with primers 27f (5' - TTTCTGTTGGTGCTGATATTGCAGRGTTYGATYMTGGCTCAG - 3' (SEQ ID NO:1)) and 1492r (5' - ACTTGCCTGTCGCTCTATCTTCCGGTTACCTTGTTACGACTT - 3' (SEQ ID NO:2)). The 22 nt 5' tails were added to the primers to serve as the annealing sites for the barcoding primers. The PCRs were carried out in 12.5 $\mu$l total volume containing 6.25 $\mu$l LongAmp Taq 2X Master Mix (New England Biolabs, Ipswich MA, USA), the primers in 400 nM concentration, and 0.1 ng template DNA. The reactions were run in a T100 thermal cycler (BioRad, Hercules CA, USA). The initial denaturation at 95 °C for 4 minutes was followed by 25 cycles of 95 °C for 20 seconds, 51 °C for 30 seconds, and 65 °C for 4 minutes. The final extension was 5 minutes at 65 °C. The PCR products were check on a 2% agarose gel and cleaned with Agencourt AMPure XP (Beckman Coulter, Indianapolis IN, USA). The purity and yield of the PCR products were assessed with a NanoDrop 8000 spectrophotometer (Thermo Fisher Scientific).

Barcodes were added in a second PCR using 5'-phospated primers adapted from the Nanopore PCR barcoding expansion kit EXP-PBC096 (Oxford Nanopore Technologies, UK) **(Tables 2 and 3)**. The reactions contained in 25 $\mu$l total volume 12.5 $\mu$l LongAmp Taq 2X Master Mix, 0.5 $\mu$l barcoding primer mix, and 25 ng 16S rRNA gene PCR product. The reactions were run in a T100 thermal cycler with initial denaturation at 95 °C for 3 minutes, 12 cycles of 95 °C for 15 seconds, 62 °C for 15 seconds, and 65 °C for 4 minutes, and final extension for 15 minutes at 65 °C. PCR products were check on a 2% agarose gel, cleaned with Agencourt AMPure XP, and quantified with a NanoDrop 8000 spectrophotometer.

The barcoded samples were pooled together in a 1.5 ml tube to have a total of 500ng DNA in a final volume of 30 $\mu$l. Sequencing adapters were ligated with the NEBNext Quick Ligation Module (New England Biolabs) and the Nanopore sequencing kit SQK-LSK114: 12.5 $\mu$l Ligation buffer (LNB), 5 $\mu$l Quick T4 Ligase, and 2.5 $\mu$l adapters mix

(LA) were added to the 30 μl pool. The reaction was incubated at room temperature for 10 minutes. The library was cleaned with AMPure XP beads mix and Short Fragment Buffer (Oxfrod Nanopore Technologies). The library was sequenced on a R10.4 flow cells loading 10 fmol DNA. The flow cell was run on a GridION sequencer with real-time super accurate basecalling and a filter threshold of average Q-score 12.

**Table 2:**

| Forward barcoding primers: | | |
|---|---|---|
| Barcode | | SEQ ID NO: |
| BC01 | GGTGCTGAAGAAAGTTGTCGGTGTCTTTGTGTTAACCTTTCTGTTGGTGCTGATATTGC | 5 |
| BC02 | GGTGCTGTCGATTCCGTTTGTAGTCGTCTGTTTAACCTTTCTGTTGGTGCTGATATTGC | 6 |
| BC03 | GGTGCTGGAGTCTTGTGTCCCAGTTACCAGGTTAACCTTTCTGTTGGTGCTGATATTGC | 7 |
| BC04 | GGTGCTGTTCGGATTCTATCGTGTTTCCCTATTAACCTTTCTGTTGGTGCTGATATTGC | 8 |
| BC05 | GGTGCTGCTTGTCCAGGGTTTGTGTAACCTTTTAACCTTTCTGTTGGTGCTGATATTGC | 9 |
| BC06 | GGTGCTGTTCTCGCAAAGGCAGAAAGTAGTCTTAACCTTTCTGTTGGTGCTGATATTGC | 10 |
| BC07 | GGTGCTGGTGTTACCGTGGGAATGAATCCTTTTAACCTTTCTGTTGGTGCTGATATTGC | 11 |
| BC08 | GGTGCTGTTCAGGGAACAAACCAAGTTACGTTTAACCTTTCTGTTGGTGCTGATATTGC | 12 |
| BC09 | GGTGCTGAACTAGGCACAGCGAGTCTTGGTTTTAACCTTTCTGTTGGTGCTGATATTGC | 13 |
| BC10 | GGTGCTGAAGCGTTGAAACCTTTGTCCTCTCTTAACCTTTCTGTTGGTGCTGATATTGC | 14 |
| BC11 | GGTGCTGGTTTCATCTATCGGAGGGAATGGATTAACCTTTCTGTTGGTGCTGATATTGC | 15 |
| BC12 | GGTGCTGCAGGTAGAAAGAAGCAGAATCGGATTAACCTTTCTGTTGGTGCTGATATTGC | 16 |
| BC13 | GGTGCTGAGAACGACTTCCATACTCGTGTGATTAACCTTTCTGTTGGTGCTGATATTGC | 17 |
| BC14 | GGTGCTGAACGAGTCTCTTGGGACCCATAGATTAACCTTTCTGTTGGTGCTGATATTGC | 18 |
| BC15 | GGTGCTGAGGTCTACCTCGCTAACACCACTGTTAACCTTTCTGTTGGTGCTGATATTGC | 19 |
| BC16 | GGTGCTGCGTCAACTGACAGTGGTTCGTACTTAACCTTTCTGTTGGTGCTGATATTGC | 20 |
| BC17 | GGTGCTGACCCTCCAGGAAAGTACCTCTGATTAACCTTTCTGTTGGTGCTGATATTGC | 21 |
| BC18 | GGTGCTGCCAAACCCAACAACCTAGATAGGCTTAACCTTTCTGTTGGTGCTGATATTGC | 22 |
| BC19 | GGTGCTGGTTCCTCGTGCAGTGTCAAGAGATTTAACCTTTCTGTTGGTGCTGATATTGC | 23 |
| BC20 | GGTGCTGTTGCGTCCTGTTACGAGAACTCATTTAACCTTTCTGTTGGTGCTGATATTGC | 24 |
| BC21 | GGTGCTGGAGCCTCTCATTGTCCGTTCTCTATTAACCTTTCTGTTGGTGCTGATATTGC | 25 |
| BC22 | GGTGCTGACCACTGCCATGTATCAAAGTACGTTAACCTTTCTGTTGGTGCTGATATTGC | 26 |
| BC23 | GGTGCTGCTTACTACCCAGTGAACCTCCTCGTTAACCTTTCTGTTGGTGCTGATATTGC | 27 |
| BC24 | GGTGCTGGCATAGTTCTGCATGATGGGTTAGTTAACCTTTCTGTTGGTGCTGATATTGC | 28 |
| BC25 | GGTGCTGGTAAGTTGGGTATGCAACGCAATGTTAACCTTTCTGTTGGTGCTGATATTGC | 29 |
| BC26 | GGTGCTGCATACAGCGACTACGCATTCTCATTTAACCTTTCTGTTGGTGCTGATATTGC | 30 |
| BC27 | GGTGCTGCGACGGTTAGATTCACCTCTTACATTAACCTTTCTGTTGGTGCTGATATTGC | 31 |
| BC28 | GGTGCTGTGAAACCTAAGAAGGCACCGTATCTTAACCTTTCTGTTGGTGCTGATATTGC | 32 |
| BC29 | GGTGCTGCTAGACACCTTGGGTTGACAGACCTTAACCTTTCTGTTGGTGCTGATATTGC | 33 |
| BC30 | GGTGCTGTCAGTGAGGATCTACTTCGACCCATTAACCTTTCTGTTGGTGCTGATATTGC | 34 |
| BC31 | GGTGCTGTGCGTACAGCAATCAGTTACATTGTTAACCTTTCTGTTGGTGCTGATATTGC | 35 |
| BC32 | GGTGCTGCCAGTAGAAGTCCGACAACGTCATTTAACCTTTCTGTTGGTGCTGATATTGC | 36 |

(continued)

| Forward barcoding primers: | | |
|---|---|---|
| Barcode | | SEQ ID NO: |
| BC33 | GGTGCTGCAGACTTGGTACGGTTGGGTAACTTTAACCTTTCTGTTGGTGCTGATATTGC | 37 |
| BC34 | GGTGCTGGGACGAAGAACTCAAGTCAAAGGCTTAACCTTTCTGTTGGTGCTGATATTGC | 38 |
| BC35 | GGTGCTGCTACTTACGAAGCTGAGGGACTGCTTAACCTTTCTGTTGGTGCTGATATTGC | 39 |
| BC36 | GGTGCTGATGTCCCAGTTAGAGGAGGAAACATTAACCTTTCTGTTGGTGCTGATATTGC | 40 |
| BC37 | GGTGCTGGCTTGCGATTGATGCTTAGTATCATTAACCTTTCTGTTGGTGCTGATATTGC | 41 |
| BC38 | GGTGCTGACCACAGGAGGACGATACAGAGAATTAACCTTTCTGTTGGTGCTGATATTGC | 42 |
| BC39 | GGTGCTGCCACAGTGTCAACTAGAGCCTCTCTTAACCTTTCTGTTGGTGCTGATATTGC | 43 |
| BC40 | GGTGCTGTAGTTTGGATGACCAAGGATAGCCTTAACCTTTCTGTTGGTGCTGATATTGC | 44 |
| BC41 | GGTGCTGGGAGTTCGTCCAGAGAAGTACACGTTAACCTTTCTGTTGGTGCTGATATTGC | 45 |
| BC42 | GGTGCTGCTACGTGTAAGGCATACCTGCCAGTTAACCTTTCTGTTGGTGCTGATATTGC | 46 |
| BC43 | GGTGCTGCTTTCGTTGTTGACTCGACGGTAGTTAACCTTTCTGTTGGTGCTGATATTGC | 47 |
| BC44 | GGTGCTGAGTAGAAAGGGTTCCTTCCCACTCTTAACCTTTCTGTTGGTGCTGATATTGC | 48 |
| BC45 | GGTGCTGGATCCAACAGAGATGCCTTCAGTGTTAACCTTTCTGTTGGTGCTGATATTGC | 49 |
| BC46 | GGTGCTGGCTGTGTTCCACTTCATTCTCCTGTTAACCTTTCTGTTGGTGCTGATATTGC | 50 |
| BC47 | GGTGCTGGTGCAACTTTCCCACAGGTAGTTCTTAACCTTTCTGTTGGTGCTGATATTGC | 51 |
| BC48 | GGTGCTGCATCTGGAACGTGGTACACCTGTATTAACCTTTCTGTTGGTGCTGATATTGC | 52 |
| BC49 | GGTGCTGACTGGTGCAGCTTTGAACATCTAGTTAACCTTTCTGTTGGTGCTGATATTGC | 53 |
| BC50 | GGTGCTGATGGACTTTGGTAACTTCCTGCGTTTAACCTTTCTGTTGGTGCTGATATTGC | 54 |
| BC51 | GGTGCTGGTTGAATGAGCCTACTGGGTCCTCTTAACCTTTCTGTTGGTGCTGATATTGC | 55 |
| BC52 | GGTGCTGTGAGAGACAAGATTGTTCGTGGACTTAACCTTTCTGTTGGTGCTGATATTGC | 56 |
| BC53 | GGTGCTGAGATTCAGACCGTCTCATGCAAAGTTAACCTTTCTGTTGGTGCTGATATTGC | 57 |
| BC54 | GGTGCTGCAAGAGCTTTGACTAAGGAGCATGTTAACCTTTCTGTTGGTGCTGATATTGC | 58 |
| BC55 | GGTGCTGTGGAAGATGAGACCCTGATCTACGTTAACCTTTCTGTTGGTGCTGATATTGC | 59 |
| BC56 | GGTGCTGTCACTACTCAACAGGTGGCATGAATTAACCTTTCTGTTGGTGCTGATATTGC | 60 |
| BC57 | GGTGCTGGCTAGGTCAATCTCCTTCGGAAGTTTAACCTTTCTGTTGGTGCTGATATTGC | 61 |
| BC58 | GGTGCTGCAGGTTACTCCTCCGTGAGTCTGATTAACCTTTCTGTTGGTGCTGATATTGC | 62 |
| BC59 | GGTGCTGTCAATCAAGAAGGGAAAGCAAGGTTTAACCTTTCTGTTGGTGCTGATATTGC | 63 |
| BC60 | GGTGCTGCATGTTCAACCAAGGCTTCTATGGTTAACCTTTCTGTTGGTGCTGATATTGC | 64 |
| BC61 | GGTGCTGAGAGGGTACTATGTGCCTCAGCACTTAACCTTTCTGTTGGTGCTGATATTGC | 65 |
| BC62 | GGTGCTGCACCCACACTTACTTCAGGACGTATTAACCTTTCTGTTGGTGCTGATATTGC | 66 |
| BC63 | GGTGCTGTTCTGAAGTTCCTGGGTCTTGAACTTAACCTTTCTGTTGGTGCTGATATTGC | 67 |
| BC64 | GGTGCTGGACAGACACCGTTCATCGACTTTCTTAACCTTTCTGTTGGTGCTGATATTGC | 68 |
| BC65 | GGTGCTGTTCTCAGTCTTCCTCCAGACAAGGTTAACCTTTCTGTTGGTGCTGATATTGC | 69 |
| BC66 | GGTGCTGCCGATCCTTGTGGCTTCTAACTTCTTAACCTTTCTGTTGGTGCTGATATTGC | 70 |
| BC67 | GGTGCTGGTTTGTCATACTCGTGTGCTCACCTTAACCTTTCTGTTGGTGCTGATATTGC | 71 |

(continued)

| | Forward barcoding primers: | |
|---|---|---|
| Barcode | | SEQ ID NO: |
| BC68 | GGTGCTGGAATCTAAGCAAACACGAAGGTGGTTAACCTTTCTGTTGGTGCTGATATTGC | 72 |
| BC69 | GGTGCTGTACAGTCCGAGCCTCATGTGATCTTTAACCTTTCTGTTGGTGCTGATATTGC | 73 |
| BC70 | GGTGCTGACCGAGATCCTACGAATGGAGTGTTTAACCTTTCTGTTGGTGCTGATATTGC | 74 |
| BC71 | GGTGCTGCCTGGGAGCATCAGGTAGTAACAGTTAACCTTTCTGTTGGTGCTGATATTGC | 75 |
| BC72 | GGTGCTGTAGCTGACTGTCTTCCATACCGACTTAACCTTTCTGTTGGTGCTGATATTGC | 76 |
| BC73 | GGTGCTGAAGAAACAGGATGACAGAACCCTCTTAACCTTTCTGTTGGTGCTGATATTGC | 77 |
| BC74 | GGTGCTGTACAAGCATCCCAACACTTCCACTTTAACCTTTCTGTTGGTGCTGATATTGC | 78 |
| BC75 | GGTGCTGGACCATTGTGATGAACCCTGTTGTTTAACCTTTCTGTTGGTGCTGATATTGC | 79 |
| BC76 | GGTGCTGATGCTTGTTACATCAACCCTGGACTTAACCTTTCTGTTGGTGCTGATATTGC | 80 |
| BC77 | GGTGCTGCGACCTGTTTCTCAGGGATACAACTTAACCTTTCTGTTGGTGCTGATATTGC | 81 |
| BC78 | GGTGCTGAACAACCGAACCTTTGAATCAGAATTAACCTTTCTGTTGGTGCTGATATTGC | 82 |
| BC79 | GGTGCTGTCTCGGAGATAGTTCTCACTGCTGTTAACCTTTCTGTTGGTGCTGATATTGC | 83 |
| BC80 | GGTGCTGCGGATGAACATAGGATAGCGATTCTTAACCTTTCTGTTGGTGCTGATATTGC | 84 |
| BC81 | GGTGCTGCCTCATCTTGTGAAGTTGTTTCGGTTAACCTTTCTGTTGGTGCTGATATTGC | 85 |
| BC82 | GGTGCTGACGGTATGTCGAGTTCCAGGACTATTAACCTTTCTGTTGGTGCTGATATTGC | 86 |
| BC83 | GGTGCTGTGGCTTGATCTAGGTAAGGTCGAATTAACCTTTCTGTTGGTGCTGATATTGC | 87 |
| BC84 | GGTGCTGGTAGTGGACCTAGAACCTGTGCCATTAACCTTTCTGTTGGTGCTGATATTGC | 88 |
| BC85 | GGTGCTGAACGGAGGAGTTAGTTGGATGATCTTAACCTTTCTGTTGGTGCTGATATTGC | 89 |
| BC86 | GGTGCTGAGGTGATCCCAACAAGCGTAAGTATTAACCTTTCTGTTGGTGCTGATATTGC | 90 |
| BC87 | GGTGCTGTACATGCTCCTGTTGTTAGGGAGGTTAACCTTTCTGTTGGTGCTGATATTGC | 91 |
| BC88 | GGTGCTGTCTTCTACTACCGATCCGAAGCAGTTAACCTTTCTGTTGGTGCTGATATTGC | 92 |
| BC89 | GGTGCTGACAGCATCAATGTTTGGCTAGTTGTTAACCTTTCTGTTGGTGCTGATATTGC | 93 |
| BC90 | GGTGCTGGATGTAGAGGGTACGGTTTGAGGCTTAACCTTTCTGTTGGTGCTGATATTGC | 94 |
| BC91 | GGTGCTGGGCTCCATAGGAACTCACGCTACTTTAACCTTTCTGTTGGTGCTGATATTGC | 95 |
| BC92 | GGTGCTGTTGTGAGTGGAAAGATACAGGACCTTAACCTTTCTGTTGGTGCTGATATTGC | 96 |
| BC93 | GGTGCTGAGTTTCCATCACTTCAGACTTGGGTTAACCTTTCTGTTGGTGCTGATATTGC | 97 |
| BC94 | GGTGCTGGATTGTCCTCAAACTGCCACCTACTTAACCTTTCTGTTGGTGCTGATATTGC | 98 |
| BC95 | GGTGCTGCCTGTCTGGAAGAAGAATGGACTTTTAACCTTTCTGTTGGTGCTGATATTGC | 99 |
| BC96 | GGTGCTGCTGAACGGTCATAGAGTCCACCATTTAACCTTTCTGTTGGTGCTGATATTGC | 100 |

In the above table, the 5'flank is represented by the first 7 nucleotides, the barcode is represented by the subsequent 24 nucleotides, the 3' flank by the subsequent 6 nucleotides and the tail by the last 22 nucleotides. All the above primers are 5'-phosphated.

**Table 3:**

| Reverse barcoding primers: | | |
|---|---|---|
| Barcode | | SEQ ID NO: |
| BC01 | GGTGCTGAAGAAAGTTGTCGGTGTCTTTGTGTTAACCTACTTGCCTGTCGCTCTATCTTC | 101 |
| BC02 | GGTGCTGTCGATTCCGTTTGTAGTCGTCTGTTTAACCTACTTGCCTGTCGCTCTATCTTC | 102 |
| BC03 | GGTGCTGGAGTCTTGTGTCCCAGTTACCAGGTTAACCTACTTGCCTGTCGCTCTATCTTC | 103 |
| BC04 | GGTGCTGTTCGGATTCTATCGTGTTTCCCTATTAACCTACTTGCCTGTCGCTCTATCTTC | 104 |
| BC05 | GGTGCTGCTTGTCCAGGGTTTGTGTAACCTTTTAACCTACTTGCCTGTCGCTCTATCTTC | 105 |
| BC06 | GGTGCTGTTCTCGCAAAGGCAGAAAGTAGTCTTAACCTACTTGCCTGTCGCTCTATCTTC | 106 |
| BC07 | GGTGCTGGTGTTACCGTGGGAATGAATCCTTTTAACCTACTTGCCTGTCGCTCTATCTTC | 107 |
| BC08 | GGTGCTGTTCAGGGAACAAACCAAGTTACGTTTAACCTACTTGCCTGTCGCTCTATCTTC | 108 |
| BC09 | GGTGCTGAACTAGGCACAGCGAGTCTTGGTTTTAACCTACTTGCCTGTCGCTCTATCTTC | 109 |
| BC10 | GGTGCTGAAGCGTTGAAACCTTTGTCCTCTCTTAACCTACTTGCCTGTCGCTCTATCTTC | 110 |
| BC11 | GGTGCTGGTTTCATCTATCGGAGGGAATGGATTAACCTACTTGCCTGTCGCTCTATCTTC | 111 |
| BC12 | GGTGCTGCAGGTAGAAAGAAGCAGAATCGGATTAACCTACTTGCCTGTCGCTCTATCTTC | 112 |
| BC13 | GGTGCTGAGAACGACTTCCATACTCGTGTGATTAACCTACTTGCCTGTCGCTCTATCTTC | 113 |
| BC14 | GGTGCTGAACGAGTCTCTTGGGACCCATAGATTAACCTACTTGCCTGTCGCTCTATCTTC | 114 |
| BC15 | GGTGCTGAGGTCTACCTCGCTAACACCACTGTTAACCTACTTGCCTGTCGCTCTATCTTC | 115 |
| BC16 | GGTGCTGCGTCAACTGACAGTGGTTCGTACTTTAACCTACTTGCCTGTCGCTCTATCTTC | 116 |
| BC17 | GGTGCTGACCCTCCAGGAAAGTACCTCTGATTTAACCTACTTGCCTGTCGCTCTATCTTC | 117 |
| BC18 | GGTGCTGCCAAACCCAACAACCTAGATAGGCTTAACCTACTTGCCTGTCGCTCTATCTTC | 118 |
| BC19 | GGTGCTGGTTCCTCGTGCAGTGTCAAGAGATTAACCTACTTGCCTGTCGCTCTATCTTC | 119 |
| BC20 | GGTGCTGTTGCGTCCTGTTACGAGAACTCATTTAACCTACTTGCCTGTCGCTCTATCTTC | 120 |
| BC21 | GGTGCTGGAGCCTCTCATTGTCCGTTCTCTATTAACCTACTTGCCTGTCGCTCTATCTTC | 121 |
| BC22 | GGTGCTGACCACTGCCATGTATCAAAGTACGTTAACCTACTTGCCTGTCGCTCTATCTTC | 122 |
| BC23 | GGTGCTGCTTACTACCCAGTGAACCTCCTCGTTAACCTACTTGCCTGTCGCTCTATCTTC | 123 |
| BC24 | GGTGCTGGCATAGTTCTGCATGATGGGTTAGTTAACCTACTTGCCTGTCGCTCTATCTTC | 124 |

(continued)

| Barcode | Reverse barcoding primers: | SEQ ID NO: |
|---|---|---|
| BC25 | GGTGCTGGTAAGTTGGGTATGCAACGCAATGTTAACCTACTTGCCTGTCGCTCTATCTTC | 125 |
| BC26 | GGTGCTGCATACAGCGACTACGCATTCTCACATTAACCTACTTGCCTGTCGCTCTATCTTC | 126 |
| BC27 | GGTGCTGCGACGGTTAGATTCACCTCTTACATTAACCTACTTGCCTGTCGCTCTATCTTC | 127 |
| BC28 | GGTGCTGTGAAACCTAAGAAGGCACCGTATCTTAACCTACTTGCCTGTCGCTCTATCTTC | 128 |
| BC29 | GGTGCTGCTAGACACCTTGGGTTGACAGACCTTAACCTACTTGCCTGTCGCTCTATCTTC | 129 |
| BC30 | GGTGCTGTCAGTGAGGATCTACTTCGACCCATTAACCTACTTGCCTGTCGCTCTATCTTC | 130 |
| BC31 | GGTGCTGTGCGTACAGCAATCAGTTACATTGTTAACCTACTTGCCTGTCGCTCTATCTTC | 131 |
| BC32 | GGTGCTGCCAGTAGAAGTCCGACAACGTCATTTAACCTACTTGCCTGTCGCTCTATCTTC | 132 |
| BC33 | GGTGCTGCAGACTTGGTACGGTTGGGTAACTTTAACCTACTTGCCTGTCGCTCTATCTTC | 133 |
| BC34 | GGTGCTGGGACGAAGAACTCAAGTCAAAGGCTTAACCTACTTGCCTGTCGCTCTATCTTC | 134 |
| BC35 | GGTGCTGCTACTTACGAAGCTGAGGGACTGCTTAACCTACTTGCCTGTCGCTCTATCTTC | 135 |
| BC36 | GGTGCTGATGTCCCAGTTAGAGGAGGAGAAACATTAACCTACTTGCCTGTCGCTCTATCTTC | 136 |
| BC37 | GGTGCTGGCTTGCGATTGATGCTTAGTATCATTAACCTACTTGCCTGTCGCTCTATCTTC | 137 |
| BC38 | GGTGCTGACCACAGGAGGACGATACAGAGAATTAACCTACTTGCCTGTCGCTCTATCTTC | 138 |
| BC39 | GGTGCTGCCACAGTGTCAACTAGAGCCTCTCTTAACCTACTTGCCTGTCGCTCTATCTTC | 139 |
| BC40 | GGTGCTGTAGTTTGGATGACCAAGGATAGCCTTAACCTACTTGCCTGTCGCTCTATCTTC | 140 |
| BC41 | GGTGCTGGGAGTTCGTCCAGAGAAGTACACGTTAACCTACTTGCCTGTCGCTCTATCTTC | 141 |
| BC42 | GGTGCTGCTACGTGTAAGGCATACCTGCCAGTTAACCTACTTGCCTGTCGCTCTATCTTC | 142 |
| BC43 | GGTGCTGCTTTCGTTGTTGACTCGACGGTAGTTAACCTACTTGCCTGTCGCTCTATCTTC | 143 |
| BC44 | GGTGCTGAGTAGAAAGGGTTCCTTCCCACTCTTAACCTACTTGCCTGTCGCTCTATCTTC | 144 |
| BC45 | GGTGCTGGATCCAACAGAGATGCCTTCAGTGTTAACCTACTTGCCTGTCGCTCTATCTTC | 145 |
| BC46 | GGTGCTGGCTGTGTTCCACTTCATTCTCCTGTTAACCTACTTGCCTGTCGCTCTATCTTC | 146 |
| BC47 | GGTGCTGGTGCAACTTTCCCACAGGTAGTTCTTAACCTACTTGCCTGTCGCTCTATCTTC | 147 |
| BC48 | GGTGCTGCATCTGGAACGTGGTACACCTGTATTAACCTACTTGCCTGTCGCTCTATCTTC | 148 |

24

| Reverse barcoding primers: | | |
|---|---|---|
| Barcode | | SEQ ID NO: |
| BC49 | GGTGCTGACTGGTGCAGCTTTGAACATCTAGTTAACCTACTTGCCTGTCGCTCTATCTTC | 149 |
| BC50 | GGTGCTGATGGACTTTGGTAACTTCCTGCGTTTAACCTACTTGCCTGTCGCTCTATCTTC | 150 |
| BC51 | GGTGCTGGTTGAATGAGCCTACTGGGTCCTCTTAACCTACTTGCCTGTCGCTCTATCTTC | 151 |
| BC52 | GGTGCTGTGAGAGACAAGATTGTTCGTGGACTTAACCTACTTGCCTGTCGCTCTATCTTC | 152 |
| BC53 | GGTGCTGAGATTCAGACCGTCTCATGCAAAGTTAACCTACTTGCCTGTCGCTCTATCTTC | 153 |
| BC54 | GGTGCTGCAAGAGCTTTGACTAAGGAGCATGTTAACCTACTTGCCTGTCGCTCTATCTTC | 154 |
| BC55 | GGTGCTGTGGAAGATGAGACCCTGATCTACGTTAACCTACTTGCCTGTCGCTCTATCTTC | 155 |
| BC56 | GGTGCTGTCACTACTCAACAGGTGGCATGAATTAACCTACTTGCCTGTCGCTCTATCTTC | 156 |
| BC57 | GGTGCTGGCTAGGTCAATCTCCTTCGGAAGTTTAACCTACTTGCCTGTCGCTCTATCTTC | 157 |
| BC58 | GGTGCTGCAGGTTACTCCTCCGTGAGTCTGATTAACCTACTTGCCTGTCGCTCTATCTTC | 158 |
| BC59 | GGTGCTGTCAATCAAGAAGGGAAAGCAAGGTTTAACCTACTTGCCTGTCGCTCTATCTTC | 159 |
| BC60 | GGTGCTGCATGTTCAACCAAGGCTTCTATGGTTAACCTACTTGCCTGTCGCTCTATCTTC | 160 |
| BC61 | GGTGCTGAGAGGGTACTATGTGCCTCAGCACTTAACCTACTTGCCTGTCGCTCTATCTTC | 161 |
| BC62 | GGTGCTGCACCCACACTTACTTCAGGACGTATTAACCTACTTGCCTGTCGCTCTATCTTC | 162 |
| BC63 | GGTGCTGTTCTGAAGTTCCTGGGTCTTGAACTTAACCTACTTGCCTGTCGCTCTATCTTC | 163 |
| BC64 | GGTGCTGGACAGACACCGTTCATCGACTTTCTTAACCTACTTGCCTGTCGCTCTATCTTC | 164 |
| BC65 | GGTGCTGTTCTCAGTCTTCCTCCAGACAAGGTTAACCTACTTGCCTGTCGCTCTATCTTC | 165 |
| BC66 | GGTGCTGCCGATCCTTGTGGCTTCTAACTTCTTAACCTACTTGCCTGTCGCTCTATCTTC | 166 |
| BC67 | GGTGCTGGTTTGTCATACTCGTGTGCTCACCTTAACCTACTTGCCTGTCGCTCTATCTTC | 167 |
| BC68 | GGTGCTGGAATCTAAGCAAACACGAAGGTGGTTAACCTACTTGCCTGTCGCTCTATCTTC | 168 |
| BC69 | GGTGCTGTACAGTCCGAGCCTCATGTGATCTTTAACCTACTTGCCTGTCGCTCTATCTTC | 169 |
| BC70 | GGTGCTGACCGAGATCCTACGAATGGAGTGTTTAACCTACTTGCCTGTCGCTCTATCTTC | 170 |
| BC71 | GGTGCTGCCTGGGAGCATCAGGTAGTAACAGTTAACCTACTTGCCTGTCGCTCTATCTTC | 171 |
| BC72 | GGTGCTGTAGCTGACTGTCTTCCATACCGACTTAACCTACTTGCCTGTCGCTCTATCTTC | 172 |

| Reverse barcoding primers: | | |
|---|---|---|
| Barcode | | SEQ ID NO: |
| BC73 | GGTGCTGAAGAAACAGGATGACAGAACCCTCTTAACCTACTTGCCTGTCGCTCTATCTTC | 173 |
| BC74 | GGTGCTGTACAAGCATCCCAACACTTCCACTTTAACCTACTTGCCTGTCGCTCTATCTTC | 174 |
| BC75 | GGTGCTGGACCATTGTGATGAACCCTGTTGTTAACCTACTTGCCTGTCGCTCTATCTTC | 175 |
| BC76 | GGTGCTGATGCTTGTTACATCAACCCTGGACTTAACCTACTTGCCTGTCGCTCTATCTTC | 176 |
| BC77 | GGTGCTGCGACCTGTTTCTCAGGGATACAACTTAACCTACTTGCCTGTCGCTCTATCTTC | 177 |
| BC78 | GGTGCTGAACAACCGAACCTTTGAATCAGAATTAACCTACTTGCCTGTCGCTCTATCTTC | 178 |
| BC79 | GGTGCTGTCTCGGAGATAGTTCTCACTGCTGTTAACCTACTTGCCTGTCGCTCTATCTTC | 179 |
| BC80 | GGTGCTGCGGATGAACATAGGATAGCGATTCTTAACCTACTTGCCTGTCGCTCTATCTTC | 180 |
| BC81 | GGTGCTGCCTCATCTTGTGAAGTTGTTTCGGTTAACCTACTTGCCTGTCGCTCTATCTTC | 181 |
| BC82 | GGTGCTGACGGTATGTCGAGTTCCAGGACTATTAACCTACTTGCCTGTCGCTCTATCTTC | 182 |
| BC83 | GGTGCTGTGGCTTGATCTAGGTAAGGTCGAATTAACCTACTTGCCTGTCGCTCTATCTTC | 183 |
| BC84 | GGTGCTGGTAGTGGACCTAGAACCTGTGCCATTAACCTACTTGCCTGTCGCTCTATCTTC | 184 |
| BC85 | GGTGCTGAACGGAGGAGTTAGTTGGATGATCTTAACCTACTTGCCTGTCGCTCTATCTTC | 185 |
| BC86 | GGTGCTGAGGTGATCCCAACAAGCGTAAGTATTAACCTACTTGCCTGTCGCTCTATCTTC | 186 |
| BC87 | GGTGCTGTACATGCTCCTGTTGTTAGGGAGGTTAACCTACTTGCCTGTCGCTCTATCTTC | 187 |
| BC88 | GGTGCTGTCTTCTACTACCGATCCGAAGCAGTTAACCTACTTGCCTGTCGCTCTATCTTC | 188 |
| BC89 | GGTGCTGACAGCATCAATGTTTGGCTAGTTGTTAACCTACTTGCCTGTCGCTCTATCTTC | 189 |
| BC90 | GGTGCTGGATGTAGAGGGTACGGTTTGAGGCTTAACCTACTTGCCTGTCGCTCTATCTTC | 190 |
| BC91 | GGTGCTGGGCTCCATAGGAACTCACGCTACTTTAACCTACTTGCCTGTCGCTCTATCTTC | 191 |
| BC92 | GGTGCTGTTGTGAGTGGAAAGATACAGGACCTTAACCTACTTGCCTGTCGCTCTATCTTC | 192 |
| BC93 | GGTGCTGAGTTTCCATCACTTCAGACTTGGGTTAACCTACTTGCCTGTCGCTCTATCTTC | 193 |
| BC94 | GGTGCTGGATTGTCCTCAAACTGCCACCTACTTAACCTACTTGCCTGTCGCTCTATCTTC | 194 |
| BC95 | GGTGCTGCCTGTCTGGAAGAAGAATGGACTTTTAACCTACTTGCCTGTCGCTCTATCTTC | 195 |
| BC96 | GGTGCTGCTGAACGGTCATAGAGTCCACCATTTAACCTACTTGCCTGTCGCTCTATCTTC | 196 |

In the above table, the 5'flank is represented by the first 7 nucleotides, the barcode is represented by the subsequent 24 nucleotides, the 3' flank by the subsequent 7 nucleotides and the tail by the last 22 nucleotides. All the above primers are 5'-phosphated.

**Data processing**

MiTRA is a bioinformatic tool that wraps many other software packages, with the aim of streamlining the microbiome composition analysis, interpreting raw DNA sequencing data from NGS and translating them into easy-to-understand test reports.

The pipeline is composed of:

- Linux Ubuntu 22.04 with:

  - Conda v4.12.0

  - Cutadapt v3.5

    i. Python 3.9.10

  - Yacrd v1.0.0

    i. Bzip2 v1.0.8
    ii. Libgcc-ng v1.2.13
    iii. Libzlib v1.2.12
    iv. Xz v5.2.5

  - Minimap2 v2.24

    i. K8 v0.2.5
    ii. Libgcc-ng v12.1.0
    iii. Libzlib v1.2.12
    iv. Zlib v1.2.12

  - R 4.2.1 with:

    i. Dada2 v4.2.1
    ii. Data.table v1.14.2
    iii. DoBy v4.6.13
    iv. Dplyr v1.0.9
    v. Openxlsx v4.2.5
    vi. Phyloseq v1.40.0
    vii. Readr v2.1.2
    viii. Reshape v0.8.9
    ix. Reshape2 v1.4.4
    x. Tidyr v1.2.0
    xi. Ridyverse v1.3.2

- Microsoft Windows 10 with:

  - R 4.2.1 (packaged in MiTRA.zip) with:

    i. htmltools
    ii. magrittr
    iii. readr
    iv. readxl
    v. rlang
    vi. shiny
    vii. shinyBS

viii. shiny Widgets
ix. shinyj s
x. tools
xi. golem
xii. devtools
xiii. MiTRA

- Google Chrome

MiTRA is designed to take users from raw sequencing data generated with Oxford Nanopore Technology or other platforms (FASTQ files) through publication quality graphics and statistics. This includes demultiplexing and quality filtering, OTU picking, taxonomic assignment, phylogenetic reconstruction, diversity analyses and visualizations.
The tool includes the following steps:

**Pre-processing and Taxonomic assignation pipeline**

The pipeline was designed to automatically process raw sequencing data inferring microbial taxonomy.

1. Trimming amplicon extremities to remove synthetic primer binding sites
2. Filtering retaining reads between 1,300 and 1,600 nucleotides in length
3. Removing low quality reads limiting spurious species detections
4. Detecting and filtering chimeric reads (chimeras)
5. Inferring bacterial taxonomy using a custom, manually curated reference database

**Microbiome profiling and investigation**

Community analysis

1. Create an object containing the taxonomic table (bacterial species and their corresponding counts) and the patient metadata. The terminology "counts" refers to the number of sequences detected for each specific and distinct bacterial species.
2. Filter the bacteria (optional).
3. Normalize the data (optional when analysing only one sample at the time and not comparing them to each other).
4. Merge taxa of the same taxonomic category (at the Phylum and Genus level respectively) and transform the associated counts into relative abundances.
5. Extract and report relative abundances for all phyla, genera, species and for phyla, genera, and species of interest.
6. Calculate alpha, beta diversity indexes.
7. Ordination

**Report making**

Data plotting and visualization

1. Add the information for the sample and patient to the 'Questionnaire' sheet of the resulting file.
2. Unzip 'MiTRA.zip' (this step only needs to be performed the first time).
3. Click on the 'run.vbs' file resulting from the decompression of 'MiTRA.zip'. This will run the R script and reach the local IP address at port 42069, where the app is being served. A new window with MiTRA's GUI will be displayed.
4. Click on the 'Browse' button to navigate towards and select the file for which you would like to create a report.
5. (Optional) Check the checkboxes for the optional analyses to be displayed on the report.
6. Click the 'Create Report' button. A file (html format) will be downloaded.
7. Open the file with Google Chrome.
8. Press Ctrl + P to print the file to a pdf.

**INNOVATIVE ASPECTS**

One of the main disadvantages of the Oxford Nanopore technology is its error-prone nature. The relatively high error-rate (2-5%) of Nanopore reads lead to an incorrect microbial taxonomy profiling. Tailored data analysis pipelines are needed to reduce profiling errors and spurious microbial species assignments. Raw Nanopore reads are pre-processed and quality filtered as follows. Because of 16S rRNA amplicon sequencing relies on PCR amplification, amplicons will contain synthetic sequences in the primer binding site. Primer attachment sites are trimmed with cutadapt tool. Following, quality filtering is applied keeping reads between 1,300 and 1,600 bases in length (expected full-length 16S rRNA gene) and allowing a maximum of 25 expected error per read. Read filtering is performed using the R-based dada2 tool. A well-known bias of PCR-based amplicon sequencing is the presence of fused, chimeric reads. Our internal analysis pipeline was implemented with a chimera detection and removal step relying on minimap2 and yacrd tools. In short, quality-filtered reads are mapped against a reference chimera-free database: chimeric or suspicious reads are then removed from the dataset. Taxonomy is finally inferred aligning reads against a manually curated database, containing (~134,000) microbial species-level reference sequences. To increase the accuracy of the alignment and reduce secondary alignments (species misidentification) the following precautions were used. Because of the redundant nature of the 16S rRNA gene, the number of possible minimizer occurrence was increased up to 50,000 times (allowed occurrence). 90% homology was used as percentage of minimal secondary-to-primary score ratio to output secondary mappings. Lastly, if the quality of the alignment drops too quickly (ex. errors or secondary alignments) the algorithm truncates the alignment. The output data obtained from the taxonomic assignment step consists in one unique csv file per patient, and it contains every identified bacterium, as well as their associated read counts and relative abundance. The resulting csv file format has been edited in order to be suitable for further microbial data analysis, notably due to the bacterial nomenclature formatted as one unique entity including all the different taxonomic levels. The objective of the Microbiome profiling and investigation R script is to automatically generate a simple and comprehensive output file for each patient, which includes the subject's information, as well as the composition of his gut microbiota (relative abundance, richness, etc). The first step consists in formatting the taxonomic file into an object that can be used for microbial data analysis. Briefly, a taxa table of the full taxonomic names and the associated read counts is created. Each taxonomic level is clearly identified and displayed as a unique entry in the table. A metadata table containing all the patients' relevant information (name, age, etc) is then generated. Finally, a particular object grouping both microbiota data and patient's data, called a phyloseq object, is created and used for further statistical analysis. Then the data is filtered, normalized and bacterial counts are converted into relative abundances at the Phylum level, the Genus level and the Species level. Microbial proportions are reported for both the entire community and for specific and relevant organisms. Finally, the individual gut microbial richness and evenness is assessed using alpha diversity indices.

The advantages of the present approach lie in the automation of nanopore data processing to rapidly profile and investigate the microbiota composition of large cohort of individuals.

Report making in MiTRA abides by the following concepts:

- The static content (content that is not dependent on the sample) of the report is written in HTML. Each page has its own file, plus one file each for the shared header and footer. The dynamic content (content that is dependent on the sample) is marked with the pattern '*$name_of_the_variable*';
- Static styling of the report (styling that is not dependent on the sample i.e. text alignment, column alignment, boldness of the text, etc.) is given by a stylesheet written in CSS. This file refers to the HTML file and specifies how elements should appear;
- Dynamic styling (styling that is dependent on the sample, i.e. colour dependent on "healthy/unhealthy values") is written in JavaScript and embedded into the above-mentioned HTML files;
- The front-end (UI) and the back-end (server) of the application are written in R. The app merges the pages together, reads the output from the pipeline, and substitutes the above-mentioned '*$name_of_the variable*' with the actual value thereby present. This is also true for control group values. The resulting file (.html) is downloaded;
- Opening the file in Google Chrome and printing it to pdf will result in all the styling being applied, producing the report;

All the above code is packaged in a .zip file with a portable distribution of R, Google Chrome, and a .vbs file for running the app from Windows.

**RESULTS**

**MiTRa update**

Improvements in the Oxford Nanopore chemistry and the subsequent release of new sequencing kit (Kit v14 family), further increased the per read quality of raw reads achieving $\geq$ 99% accuracy. Coupling the higher per read quality with the capability of sequencing long and ultra-long DNA fragments, made Oxford Nanopore an extremely promising tool for microbial communities profiling. Strong on that, inventors have developed and optimized a custom, in-house data analysis pipeline (herein defined as MiTRa) allowing microbial profiling based on 16S rRNA amplicon sequencing. MiTRa relies on a manually curated, species-specific database based on up-to-date nomenclature (Parte, A.C., Sardà Carbasse, J., Meier-Kolthoff, J.P., Reimer, L.C. and Göker, M. (2020). List of Prokaryotic names with Standing in Nomenclature (LPSN) moves to the DSMZ. International Journal of Systematic and Evolutionary Microbiology, 70, 5607-5612; DOI: 10.1099/ijsem.0.004332; Parks, D.H., et al. (2021). "GTDB: an ongoing census of bacterial and archaeal diversity through a phylogenetically consistent, rank normalized and complete genome-based taxonomy." Nucleic Acids Research, https://doi.org/10.1093/nar/gkab776) and minimap2 (Li, H. (2018). Minimap2: pairwise alignment for nucleotide sequences. Bioinformatics, 34:3094-3100. doi:10.1093/bioinformatics/bty191), a versatile sequence alignment tool designed to handle long and noisy Nanopore reads. MiTRa workflow includes i) trimming adapters using cutadapt (Marcel M. Cutadapt Removes Adapter Sequences From High-Throughput Sequencing Reads. EMBnet journal, 2011. https://doi.org/10.14806/ej.17.1.200) and filtering low quality reads with DADA2 (Callahan, B., McMurdie, P., Rosen, M. et al. DADA2: High-resolution sample inference from Illumina amplicon data. Nat Methods 13, 581-583 (2016). https://doi.org/10.1038/nmeth.3869), ii) filtering chimeric reads with yacrd tool (Marijon P., Chikhi R., Varré JS., yacrd and fpa: upstream tools for long-read genome assembly, Bioinformatics, btaa262, https://doi.org/10.1093/bioinformatics/btaa262) and iv) aligning quality-checked reads against the reference database inferring taxonomy. A minimum of 96% correctly matched nucleotides threshold is used to identify bacterial species and remove spurious alignments. In-house MiTRa pipeline was tested on ZymoBIOMICS Gut Microbiome Standard (Zymo Research, Irvin CA, USA), sequenced using the method disclosed in example 1 (referred as MiTRa in the table below). Results were compared to those generated by the method of example 1. Identified species and their abundances are listed in the following table (alphabetical order):

| Mock Community | | | |
|---|---|---|---|
| Species | Composition | MiTRa | Example 1 |
| *Akkermansia muciniphila* | 0.97 | 0.54 | 1.61 |
| *Bacteroides fragilis* | 9.94 | 6.97 | 13.97 |
| *Bifidobacterium adolescentis* | 8.78 | 8.28 | 6.66 |
| *Clostridioides difficile* | 2.62 | 4.79 | 5.28 |
| *Clostridium perfringens* | 0.0002 | 0 | 0 |
| *Enterococcus faecalis* | 0.0009 | 0.07 | 0 |
| *Escherichia coli* | 12.12 | 10.59 | 8.67 |
| *Faecalibacterium prausnitzii* | 17.63 | 20.94 | 0.004 |
| *Fusobacterium nucleatum* | 7.49 | 4.47 | 7.31 |
| *Limosilactobacillus fermentum* | 9.63 | 6.81 | 5.77 |
| *Roseburia hominis* | 9.89 | 8.7 | 7.07 |
| *Salmonella enterica* | 0.009 | 0.06 | 0.018 |
| | | | |

Results achieved by MiTRa are comparable to those generated by the analysis pipeline of example 1. Main differences reported are: i) proper identification of *F. prausnitizii* species, ii) improved detection of A. *muciniphila, B. adolescentis B. fragilis* and *E. coli* species, iii) detection of *E. faecalis* using MiTRa (although overestimated) and iv). In conclusion MiTRa enables a proper identification of microbial species in the mock community, although some species were overestimated. MiTRa pipeline is coupled with the up-to-date Nanopore chemistry (SQK-LSK114) as disclosed in example 2, in order to improve the accuracy of the analysis with respect to example 1.

**REFERENCES**

Abdulla, M. & Mohammed, N. A Review on Inflammatory Bowel Diseases: Recent Molecular Pathophysiology Advances. Biologies: Targets and Therapy vol. 16 129-140 Preprint at https://doi.org/10.2147BTT.S380027 (2022)).

Acharya, K., Khanal, S., Pantha, K., Amatya, N., Davenport, R. J., & Werner, D. (2019). A comparative assessment of conventional and molecular methods, including MinION nanopore sequencing, for surveying water quality. Scientific reports, 9(1), 1-11.

Ammer-Herrmenau, C., Pfisterer, N., van den Berg, T., Gavrilova, I., Amanzada, A., Singh, S. K., ... & Neesse, A. (2021). Comprehensive wet-bench and bioinformatics workflow for complex microbiota using Oxford Nanopore technologies. Msystems, 6(4), e00750-21.

Baumgart, D. C. & Sandborn, W. J. Gastroenterology 2 Inflammatory bowel disease: clinical aspects and established and evolving therapies. www.thelancet.com vol. 369 www.thelancet.com (2007)

Beale, D. J., Bissett, A., Nilsson, S., Bose, U., Nelis, J. L. D., Nahar, A., ... & Vardy, S. (2022). Perturbation of the gut microbiome in wild-caught freshwater turtles (Emydura macquarii macquarii) exposed to elevated PFAS levels. Science of The Total Environment, 156324.

Bolyen, E., Rideout, J. R., Dillon, M. R., Bokulich, N. A., Abnet, C. C., Al-Ghalith, G. A., ... & Caporaso, J. G. (2019). Reproducible, interactive, scalable and extensible microbiome data science using QIIME 2. Nature biotechnology, 37(8), 852-857.

Callahan, B. J., McMurdie, P. J., Rosen, M. J., Han, A. W., Johnson, A. J. A., & Holmes, S. P. (2016). DADA2: High-resolution sample inference from Illumina amplicon data. Nature methods, 13(7), 581-583.

Chavan, S., Sarangdhar, V., & Vigneshwaran, N. (2022). Nanopore-based metagenomic analysis of the impact of nanoparticles on soil microbial communities. Heliyon, 8(6), e09693.

Cole, J. R., Wang, Q., Fish, J. A., Chai, B., McGarrell, D. M., Sun, Y., ... & Tiedje, J. M. (2014). Ribosomal Database Project: data and tools for high throughput rRNA analysis. Nucleic acids research, 42(D1), D633-D642.

Delahaye, C., & Nicolas, J. (2021). Sequencing DNA with nanopores: Troubles and biases. PLoS One, 16(10), e0257521.

De Siqueira, G. M. V., Pereira-dos-Santos, F. M., Silva-Rocha, R., & Guazzaroni, M. E. (2021). Nanopore sequencing provides rapid and reliable insight into microbial profiles of intensive care units. Frontiers in public health, 1231.

Dragoni, G., Innocenti, T. & Galli, A. Biomarkers of Inflammation in Inflammatory Bowel Disease: How Long before Abandoning Single-Marker Approaches? Digestive Diseases 39, 190-203 (2021).

Edgar, R. C. (2010). Search and clustering orders of magnitude faster than BLAST. Bioinformatics, 26(19), 2460-2461.

Edgar, R. C., Haas, B. J., Clemente, J. C., Quince, C., & Knight, R. (2011). UCHIME improves sensitivity and speed of chimera detection. Bioinformatics, 27(16), 2194-2200.

Fujiyoshi, S., Muto-Fujita, A., & Maruyama, F. (2020). Evaluation of PCR conditions for characterizing bacterial communities with full-length 16S rRNA genes using a portable nanopore sequencer. Scientific reports, 10(1), 1-10.

Fu, Y., Chen, Q., Xiong, M., Zhao, J., Shen, S., Chen, L., ... & Li, Y. (2022). Clinical Performance of Nanopore Targeted Sequencing for Diagnosing Infectious Diseases. Microbiology Spectrum, 10(2), e00270-22.

Gevers, D. et al. The treatment-naive microbiome in new-onset Crohn's disease. Cell Host Microbe 15, 382-392 (2014).

Hanauer, S. B. Inflammatory Bowel Disease: Epidemiology, Pathogenesis, and Therapeutic Opportunities. https://academic.oup.com/ibdjournal/article/12/suppl_1/S3/4676576

Heikema, A. P., Horst-Kreft, D., Boers, S. A., Jansen, R., Hiltemann, S. D., de Koning, W., ... & Hays, J. P. (2020). Comparison of Illumina versus nanopore 16S rRNA gene sequencing of the human nasal microbiota. Genes, 11(9), 1105.

Janda, J. M., & Abbott, S. L. (2007). 16S rRNA gene sequencing for bacterial identification in the diagnostic laboratory: pluses, perils, and pitfalls. Journal of clinical microbiology, 45(9), 2761-2764.

Johnson, J. S., Spakowicz, D. J., Hong, B. Y., Petersen, L. M., Demkowicz, P., Chen, L., ... & Weinstock, G. M. (2019). Evaluation of 16S rRNA gene sequencing for species and strain-level microbiome analysis. Nature communications, 10(1), 1-11.

Kaplan, G. G. The global burden of IBD: From 2015 to 2025. Nature Reviews Gastroenterology and Hepatology vol. 12 720-727 Preprint at https://doi.org/10.1038/nrgastro.2015.150 (2015).

Kelsen, J. & Baldassano, R. N. Inflammatory bowel disease: the difference between children and adults. Inflamm Bowel Dis 14 Suppl 2, (2008).

Kinoshita, Y., Niwa, H., Uchida-Fujii, E., & Nukada, T. (2021). Establishment and assessment of an amplicon sequencing method targeting the 16S-ITS-23S rRNA operon for analysis of the equine gut microbiome. Scientific reports, 11(1), 1-12.

Kim, M., Oh, H. S., Park, S. C., & Chun, J. (2014). Towards a taxonomic coherence between average nucleotide identity and 16S rRNA gene sequence similarity for species demarcation of prokaryotes. International journal of systematic and evolutionary microbiology, 64(Pt_2), 346-351.

Kozich, J. J., Westcott, S. L., Baxter, N. T., Highlander, S. K., & Schloss, P. D. (2013).

Development of a dual-index sequencing strategy and curation pipeline for analyzing amplicon sequence data on the MiSeq Illumina sequencing platform. Applied and environmental microbiology, 79(17), 5112-5120.

Kuenzig, M. E. et al. Twenty-first Century Trends in the Global Epidemiology of Pediatric-Onset Inflammatory Bowel Disease: Systematic Review. Gastroenterology 162, 1147-1159.e4 (2022).

Li, X., Kong, P., Daughtrey, M., Kosta, K., Schirmer, S., Howle, M., ... & Hong, C. (2022). Characterization of the Soil Bacterial Community from Selected Boxwood Gardens across the United States. Microorganisms, 10(8), 1514.

Low, L., Fuentes-Utrilla, P., Hodson, J., O'Neil, J. D., Rossiter, A. E., Begum, G., ... & Rauz, S. (2021). Evaluation of full-length nanopore 16S sequencing for detection of pathogens in microbial keratitis. PeerJ, 9, e10778.

Martin, M. (2011). Cutadapt removes adapter sequences from high-throughput sequencing reads. EMBnet. journal, 17(1), 10-12.

Matsuo, Y., Komiya, S., Yasumizu, Y., Yasuoka, Y., Mizushima, K., Takagi, T., ... & Hirota, K. (2021). Full-length 16S rRNA gene amplicon analysis of human gut microbiota using MinION™ nanopore sequencing confers species-level resolution. BMC microbiology, 21(1), 1-13.

Mottawea, W. et al. Altered intestinal microbiota-host mitochondria crosstalk in new onset Crohn's disease. Nat Commun 7, (2016).

Nygaard, A. B., Tunsjø, H. S., Meisal, R., & Charnock, C. (2020). A preliminary study on the potential of Nanopore MinION and Illumina MiSeq 16S rRNA gene sequencing to characterize building-dust microbiomes. Scientific Reports, 10(1), 1-10.

Oberle, A., Urban, L., Falch-Leis, S., Ennemoser, C., Nagai, Y., Ashikawa, K., ... & Feichtinger, M. (2021). 16S rRNA long-read nanopore sequencing is feasible and reliable for endometrial microbiome analysis. Reproductive Biomedicine Online, 42(6), 1097-1107.

Oksanen J, Simpson G, Blanchet F, Kindt R, Legendre P, Minchin P, O'Hara R, Solymos P, Stevens M, Szoecs E, Wagner H, Barbour M, Bedward M, Bolker B, Borcard D, Carvalho G, Chirico M, De Caceres M, Durand S, Evangelista H, FitzJohn R, Friendly M, Furneaux B, Hannigan G, Hill M, Lahti L, McGlinn D, Ouellette M, Ribeiro Cunha E, Smith T, Stier A, Ter Braak C, Weedon J (2022). _vegan: Community Ecology Package_. R package version 2.6-2, <https://CRAN.R-project.org/package=vegan>.

Olbjorn, C., Småstuen, M. C. & Moen, A. E. F. Targeted Analysis of the Gut Microbiome for Diagnosis, Prognosis and Treatment Individualization in Pediatric Inflammatory Bowel Disease. Microorganisms 10, (2022).

Omi, M., Matsuo, Y., Araki-Sasaki, K., Oba, S., Yamada, H., Hirota, K., & Takahashi, K. (2022). 16S rRNA nanopore sequencing for the diagnosis of ocular infection: a feasibility study. BMJ Open Ophthalmology, 7(1), e000910.

Park, C., Kim, S. B., Choi, S. H., & Kim, S. (2021). Comparison of 16S rRNA gene based microbial profiling using five next-generation sequencers and various primers. Frontiers in Microbiology, 12.

Pichler, M., Coskun, Ö. K., Ortega-Arbulú, A. S., Conci, N., Wörheide, G., Vargas, S., & Orsi, W. D. (2018). A 16S rRNA gene sequencing and analysis protocol for the Illumina MiniSeq platform. Microbiologyopen, 7(6), e00611.

Pollock, J., Glendinning, L., Wisedchanwet, T., & Watson, M. (2018). The madness of microbiome: attempting to find consensus "best practice" for 16S microbiome studies. Applied and environmental microbiology, 84(7), e02627-17.

Quast, C., Pruesse, E., Yilmaz, P., Gerken, J., Schweer, T., Yarza, P., ... & Glöckner, F. O. (2012). The SILVA ribosomal RNA gene database project: improved data processing and web-based tools. Nucleic acids research, 41(D1), D590-D596.

Rognes, T., Flouri, T., Nichols, B., Quince, C., & Mahé, F. (2016). VSEARCH: a versatile open source tool for metagenomics. PeerJ, 4, e2584.

Rosen, M. J., Dhawan, A. & Saeed, S. A. Inflammatory bowel disease in children and adolescents. JAMA Pediatrics vol. 169 1053-1060 Preprint at https://doi.org/10.1001/jamapediatrics.2015.1982 (2015).

Rozas, M., Brillet, F., Callewaert, C., & Paetzold, B. (2022). MinION™ Nanopore Sequencing of Skin Microbiome 16S and 16S-23S rRNA Gene Amplicons. Frontiers in cellular and infection microbiology, 1317.

Russel, J. (2021). Russel88/MicEco: v0.9.15 (v0.9.15), Zenodo, https://doi.org/10.5281/zenodo.4733747.

Sauer, C. G. & Kugathasan, S. Pediatric Inflammatory Bowel Disease: Highlighting Pediatric Differences in IBD. Medical Clinics of North America vol. 94 35-52 Preprint at https://doi.org/10.1016/j.mcna.2009.10.002 (2010).

Schloss, P. D., Westcott, S. L., Ryabin, T., Hall, J. R., Hartmann, M., Hollister, E. B., ... & Weber, C. F. (2009). Introducing mothur: open-source, platform-independent, community-supported software for describing and comparing microbial communities. Applied and environmental microbiology, 75(23), 7537-7541.

Shin, J., Lee, S., Go, M. J., Lee, S. Y., Kim, S. C., Lee, C. H., & Cho, B. K. (2016). Analysis of the mouse gut microbiome using full-length 16S rRNA amplicon sequencing. Scientific reports, 6(1), 1-10.

Sila, S. et al. Altered Gut Microbiota Is Present in Newly Diagnosed Pediatric Patients With Inflammatory Bowel Disease. J Pediatr Gastroenterol Nutr 70, 497-502 (2020)

Silangcruz, K. et al. Impact of the World Inflammatory Bowel Disease Day and Crohn's and Colitis Awareness Week on Population Interest Between 2016 and 2020: Google Trends Analysis. JMIR Infodemiology 1, e32856 (2021).

Stahl-Rommel, S., Jain, M., Nguyen, H. N., Arnold, R. R., Aunon-Chancellor, S. M., Sharp, G. M., ... & Castro-Wallace, S. L. (2021). Real-time culture-independent microbial profiling onboard the international space station using nanopore sequencing. Genes, 12(1), 106.

Stevens, B., Creed, T., Reardon, C., & Manter, D. (2022). Comparison of Oxford Nanopore Technologies and Illumina MiSeq sequencing with mock communities and agricultural soil. doi.org/10.21203/rs.3.rs-1731798/v1

Urban, L., Holzer, A., Baronas, J. J., Hall, M. B., Braeuninger-Weimer, P., Scherm, M. J., ... & Stammnitz, M. R. (2021). Freshwater monitoring by nanopore sequencing. Elife, 10. van der Loos, L. M., D'hondt, S., Willems, A., & De Clerck, O. (2021). Characterizing algal microbiomes using long-read nanopore sequencing. Algal Research, 59, 102456.

Wei, P. L., Hung, C. S., Kao, Y. W., Lin, Y. C., Lee, C. Y., Chang, T. H., ... & Lin, J. C. (2020). Characterization of fecal microbiota with clinical specimen using long-read and short-read sequencing platform. International journal of molecular sciences, 21(19), 7110.

Winand, R., Bogaerts, B., Hoffman, S., Lefevre, L., Delvoye, M., Van Braekel, J., ... & Vanneste, K. (2019). Targeting the 16s rRNA gene for bacterial identification in complex mixed samples: Comparative evaluation of second (illumina) and third (oxford nanopore technologies) generation sequencing technologies. International journal of molecular sciences, 21(1), 298.

Yu, Y. R. & Rodriguez, J. R. Clinical presentation of Crohn's, ulcerative colitis, and indeterminate colitis: Symptoms, extraintestinal manifestations, and disease phenotypes. Semin Pediatr Surg 26, 349-355 (2017).

Zhao, M., Gönczi, L., Lakatos, P. L. & Burisch, J. The Burden of Inflammatory Bowel Disease in Europe in 2020. Journal of Crohn's and Colitis vol. 15 1573-1587 Preprint at https://doi.org/10.1093/ecco-jcc/jjab029 (2021).

## Claims

1. A method for identifying and optionally quantifying microorganisms in a sample comprising:

   (a) extracting genomic DNA from the sample to obtain an extracted genomic DNA sample ;
   (b) amplifying full-length 16S rRNA gene from the extracted genomic DNA sample to get 16S rRNA gene amplicons ;
   (c) further amplifying 16S rRNA gene amplicons generated in step (b) to ligate molecular barcodes;
   (d) Ligating adapters to the amplified amplicons of step (c) ;
   (e) Sequencing adapter-amplicons of step (d) and subsequent generating sequencing reads ;
   (f) Data analysing the sequencing reads generated in step (e) to identify, and optionally quantify, microorganisms

   wherein the method is **characterized in that**:

   i. in step (b) the amplification is carried out with primers 27f (5' - TTTCTGTTGGTGCTGATATTGCAGRGTTYGATYMTGGCTCAG - 3' (SEQ ID NO: 1)) and 1492r (5' - ACTTGCCTGTCGCTCTATCTTCCGGTTACCTTGTTACGACTT - 3' (SEQ ID NO:2)) and/or
   ii. the amplification of step (b) comprises polymerase chain reaction (PCR) and the annealing temperature of the 16S rRNA gene PCR of step (b) is 51°C and/or
   iii. the barcodes are ligated using 5'-phospated primers

   and wherein the method is capable of allowing species-level classification of the microorganisms identified or quantified in the sample.

2. The method according to claim 1 wherein the sample is a biological sample obtained from a subject, preferably the biological sample is a solid and/or liquid biological sample, such as a faecal, stool, swab, preferably nasal, skin or vaginal swab, blood, synovial fluid or respiratory wash sample, or wherein the sample is a pharmaceutical preparation.

3. The method according to claim 1 or 2, wherein the amplicons of step (b) are ~1.5 Kb in length and/or wherein the ligation of molecular barcodes of step (c) enables samples multiplexing and/or wherein the sequencing of step (e) is Nanopore sequencing, preferably Oxford Nanopore sequencing.

4. The method according to any one of previous claims wherein the further amplification is carried out with primers selected from Tables 2 and 3 and/or are listed in Tables 2 and 3.

5. The method according to any one of previous claims wherein the microorganism is a pathogen, an opportunistic and/or a commensal microorganism.

6. The method according to any one of previous claims, wherein the method identifies and/or quantify a bacterial taxa present with at least 0.01% relative abundance in the bacterial community of the samples.

7. The method according to any one of previous claims, wherein the method results in redundant detection of the microorganism at the phylum level, genus level and species level.

8. The method of any one of previous claims, wherein the amplification of step (b) and/or (c) comprises polymerase chain reaction (PCR).

9. The method according to any one of previous claims, wherein the data analysis enables i) trimming and filtering of low quality reads, ii) detection of putative chimeric reads, iii) profiling bacterial species at $\geq$ 97% accuracy and iv) generate final report including relative abundances and species of interest.

10. A method for identifying and optionally quantifying the microorganisms present in the gut microbiota or blood, or urine, or synovial fluid, or pulmonary wash sample of the subject or in sample, such as a pharmaceutical preparation, the method comprising:

   (a) providing a biological sample obtained from the subject or in the sample; and
   (b) identifying microorganism nucleic acids in the biological sample or in the sample according to the method of any one of claims 1-9.

11. A method for providing an optimized therapy for a subject based on the identification of microorganisms present in the gut microbiota of the subject carried out with the method of any one of claims 1-10, preferably wherein the optimized therapy to the patient is faster than standard of care.

12. A method for providing personalised probiotic intake, wherein the probiotic present in the personalised probiotic intake is not detected by the method of any one of claims 1-11, and is preferably one or more of the following species: *Bifidobacterium breve, Lactobacillus acidophilus, Lacticaseibacillus casei, Lactobacillus crispatus, Lactobacillus gasseri, Lacticaseibacillus paracasei, Lactiplantibacillus plantarum Limosilactobacillus reuteri, Lacticaseibacillus rhamnosus, Ligilactobacillus salivarius, Bifidobacterium longum, Bifidobacterium infantis, Bifidobacterium animalis, Lactobacillus helveticus , Bifidobacterium bifium, Lactobacillus delbrueckii* subsp. *Bulgaricus, Escherichia coli Nissle and Enterococcus faecium.*

13. A method for the prognosis and/or for determining the severity risk and/or disease development risk of an Inflammatory Bowel Disease (IBD), such as ulcerative colitis (UC) or Crohn's disease (CD), the method comprising the method of any one of claims 1-10, wherein when the method is for the prognosis and/or for determining the severity risk the subject is an IBD patient.

14. A method for the diagnosis and/or prognosis of a Dysbiosis, IBD related, or of IBD and/or for the monitoring of the efficacy of a therapeutic treatment of a Dysbiosis IBD related or of IBD and/or for the screening of a therapeutic treatment of a Dysbiosis IBD related or of IBD comprising the step of

   i. measuring a first total abundance of four or more bacterial species which are known to be increased in the IBD in a sample obtained from the subject;
   ii. measuring a second total abundance of four or more bacterial species which are known to be decreased in the IBD;
   iii. calculating the log of the ratio of the first total abundance over the second total abundance to obtain a Microbiome Index for Dysbiosis IBD related (MIDIBD)
   iv. comparing the obtained MIDIBD with reference values.

15. The method according to claim 14, wherein the species increased are selected from the group consisting of: *Ruminococcus gnavus, Bacteroides dorei, Anaerostipes hadrus, Veillonella parvula, Blautia wexlerae, Eubacterium halii* and
   wherein the species decreased are selected from butyrate producing-bacterial species, preferably they are selected from the group consisting of: *Faecalibacterium prausnitzii, Roseburia intestinalis, Clostridium leptum, Eubacterium rectale, Eubacterium hallii, Clostridium butyricum* and preferably wherein the abundance of the bacterial species is measured by carrying out the method according to any one of claims 1-10.

Fig. 1

Fig. 2

Fig. 3

Fig. 4

Europäisches
Patentamt

European
Patent Office

Office européen
des brevets

# EUROPEAN SEARCH REPORT

Application Number

EP 22 21 3544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/143158 A1 (BROAD INST INC [US]; STEELMAN SCOTT [US]; NICOL ROBERT [US]) 18 September 2014 (2014-09-18) | 1-11 | INV. C12Q1/689 |
| Y | * paragraphs [0043] - [0045], [0047], [0101] - [0108], [0114] - [0121], [0123] - [0132], [0135] - [0136], [0166] - [0175]; figures 31-35,38 * <br> * table 1 * <br> * examples 2,4-5,8 * <br> ----- | 13-15 | |
| X | AHMAD SHUKRI ZUHAYRA NASRIN ET AL: "A novel study on the effectiveness of bioflocculant-producing bacteria Bacillus enclensis, isolated from biofloc-based system as a biodegrader in microplastic pollution", CHEMOSPHERE, PERGAMON PRESS, OXFORD, GB, vol. 308, 14 September 2022 (2022-09-14), XP087192266, ISSN: 0045-6535, DOI: 10.1016/J.CHEMOSPHERE.2022.136410 [retrieved on 2022-09-14] | 1,3,8,9 | |
| Y | * the whole document * <br> * 2.6 * <br> ----- | 4-7, 10-15 | C12Q |
| X | KR 2016 0095006 A (SERES THERAPEUTICS INC [US]) 10 August 2016 (2016-08-10) | 1-3,5-8, 10-12 | |
| Y | * claims 1-244 * <br> * paragraphs [0069], [0089], [0126], [0158], [0208] - [0210], [0219], [257287] - [0289], [0297] - [0307] * <br> * examples 12, 19-24 * <br> ----- | 9,13-15 | |
| X | WO 2014/153194 A2 (SERES HEALTH INC [US]) 25 September 2014 (2014-09-25) * examples 3-10,28, 48,49 * <br> ----- | 1-3,5-15 | |

TECHNICAL FIELDS SEARCHED (IPC)

C12Q

-/--

The present search report has been drawn up for all claims

| Place of search | Date of completion of the search | Examiner |
|---|---|---|
| The Hague | 16 June 2023 | Bruma, Anja |

Europäisches
Patentamt

European
Patent Office

Office européen
des brevets

# EUROPEAN SEARCH REPORT

**Application Number**

EP 22 21 3544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROSALINDA D'AMORE ET AL: "A comprehensive benchmarking study of protocols and sequencing platforms for 16S rRNA community profiling", BMC GENOMICS, BIOMED CENTRAL LTD, LONDON, UK, vol. 17, no. 1, 14 January 2016 (2016-01-14), pages 1-20, XP021231629, DOI: 10.1186/S12864-015-2194-9 | 1-9 | |
| Y | * the whole document * | 10-15 | |
| X | CHENHAO LI ET AL: "INC-Seq: accurate single molecule reads using nanopore sequencing", GIGASCIENCE, BIOMED CENTRAL LTD, LONDON, UK, vol. 5, no. 1, 2 August 2016 (2016-08-02), pages 1-11, XP021268736, DOI: 10.1186/S13742-016-0140-7 | 1-3,5-9 | |
| Y | * the whole document * | 10-15 | TECHNICAL FIELDS SEARCHED (IPC) |
| Y | LLOYD-PRICE JASON ET AL: "Multi-omics of the gut microbial ecosystem in inflammatory bowel diseases", NATURE, NATURE PUBLISHING GROUP UK, LONDON, vol. 569, no. 7758, 29 May 2019 (2019-05-29), pages 655-662, XP036793511, ISSN: 0028-0836, DOI: 10.1038/S41586-019-1237-9 [retrieved on 2019-05-29] * the whole document * | 14,15 | |

-/--

The present search report has been drawn up for all claims

| Place of search | Date of completion of the search | Examiner |
|---|---|---|
| The Hague | 16 June 2023 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 22 21 3544

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZMORA NIV ET AL: "Personalized Gut Mucosal Colonization Resistance to Empiric Probiotics Is Associated with Unique Host and Microbiome Features", CELL, 6 September 2018 (2018-09-06), XP055809665, DOI: 10.1016/j.cell.2018.08.041 * the whole document * | 12 | |
| X | QI YING ET AL: "High-throughput sequencing provides insights into oral microbiota dysbiosis in association with inflammatory bowel disease", GENOMICS, ACADEMIC PRESS, SAN DIEGO, US, vol. 113, no. 1, 1 October 2020 (2020-10-01), pages 664-676, XP086469688, ISSN: 0888-7543, DOI: 10.1016/J.YGENO.2020.09.063 [retrieved on 2020-10-01] | 1-3,5-9 | |
| Y | * the whole document * | 13-15 | |
| Y | EP 3 130 680 A1 (UNIV GIRONA [ES] ET AL.) 15 February 2017 (2017-02-15) * claims 1-15 * * paragraph [0126]; examples 1-11 * | 1-15 | |
| X,D | GEVERS DIRK ET AL: "The Treatment-Naive Microbiome in New-Onset Crohn's Dis", CELL HOST & MICROBE, ELSEVIER, NL, vol. 15, no. 3, 12 March 2014 (2014-03-12), pages 382-392, XP028629969, ISSN: 1931-3128, DOI: 10.1016/J.CHOM.2014.02.005 | 14,15 | |
| Y | * the whole document * | 1-12 | |

TECHNICAL FIELDS SEARCHED (IPC)

The present search report has been drawn up for all claims

| Place of search | Date of completion of the search | Examiner |
|---|---|---|
| The Hague | 16 June 2023 | Bruma, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 3544

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2023

| Patent document cited in search report | | Publication date | Patent family member(s) | | Publication date |
|---|---|---|---|---|---|
| WO 2014143158 | A1 | 18-09-2014 | NONE | | |
| KR 20160095006 | A | 10-08-2016 | AU | 2014352643 A1 | 30-06-2016 |
| | | | AU | 2021200287 A1 | 18-03-2021 |
| | | | BR | 112016011830 A2 | 26-09-2017 |
| | | | CA | 2931317 A1 | 28-05-2015 |
| | | | CN | 105979952 A | 28-09-2016 |
| | | | EP | 3074027 A1 | 05-10-2016 |
| | | | JP | 6833514 B2 | 24-02-2021 |
| | | | JP | 2016537434 A | 01-12-2016 |
| | | | JP | 2021059545 A | 15-04-2021 |
| | | | KR | 20160095006 A | 10-08-2016 |
| | | | KR | 20220042246 A | 04-04-2022 |
| | | | KR | 20230047502 A | 07-04-2023 |
| | | | MX | 367109 B | 05-08-2019 |
| | | | RU | 2016125316 A | 09-01-2018 |
| | | | US | 2017151291 A1 | 01-06-2017 |
| | | | US | 2020345792 A1 | 05-11-2020 |
| | | | US | 2022257674 A1 | 18-08-2022 |
| | | | WO | 2015077794 A1 | 28-05-2015 |
| WO 2014153194 | A2 | 25-09-2014 | EP | 2971148 A2 | 20-01-2016 |
| | | | HK | 1220495 A1 | 05-05-2017 |
| | | | US | 2016040215 A1 | 11-02-2016 |
| | | | WO | 2014153194 A2 | 25-09-2014 |
| EP 3130680 | A1 | 15-02-2017 | AU | 2016306627 A1 | 15-03-2018 |
| | | | CA | 2995308 A1 | 16-02-2017 |
| | | | CN | 108474037 A | 31-08-2018 |
| | | | CN | 115976239 A | 18-04-2023 |
| | | | DK | 3334838 T3 | 15-11-2021 |
| | | | EP | 3130680 A1 | 15-02-2017 |
| | | | EP | 3334838 A1 | 20-06-2018 |
| | | | ES | 2901465 T3 | 22-03-2022 |
| | | | HK | 1257108 A1 | 11-10-2019 |
| | | | HR | P20211757 T1 | 18-02-2022 |
| | | | IL | 257461 A | 30-04-2018 |
| | | | JP | 7095210 B2 | 05-07-2022 |
| | | | JP | 2018525035 A | 06-09-2018 |
| | | | JP | 2022031644 A | 22-02-2022 |
| | | | KR | 20180048696 A | 10-05-2018 |
| | | | PT | 3334838 T | 23-11-2021 |
| | | | SI | 3334838 T1 | 31-01-2022 |
| | | | US | 2019024146 A1 | 24-01-2019 |
| | | | US | 2022307075 A1 | 29-09-2022 |
| | | | WO | 2017025617 A1 | 16-02-2017 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 22 21 3544

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2023

| Patent document cited in search report | Publication date | Patent family member(s) | Publication date |
|---|---|---|---|

---------------------------------------------------------------------

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PARTE, A.C. ; SARDÀ CARBASSE, J. ; MEI-ER-KOLTHOFF, J.P. ; REIMER, L.C. ; GÖKER, M.** List of Prokaryotic names with Standing in Nomenclature (LPSN) moves to the DSMZ. *International Journal of Systematic and Evolutionary Microbiology,* 2020, vol. 70, 5607-5612
- **PARKS, D.H. et al.** GTDB: an ongoing census of bacterial and archaeal diversity through a phylogenetically consistent, rank normalized and complete genome-based taxonomy. *Nucleic Acids Research,* 2021, https://doi.org/10.1093/nar/gkab776
- **LI, H.** Minimap2: pairwise alignment for nucleotide sequences. *Bioinformatics,* 2018, vol. 34, 3094-3100
- **MARCEL M.** Cutadapt Removes Adapter Sequences From High-Throughput Sequencing Reads. *EMBnet journal,* 2011, https://doi.org/10.14806/ej.17.1.200
- **CALLAHAN, B. ; MCMURDIE, P. ; ROSEN, M. et al.** DADA2: High-resolution sample inference from Illumina amplicon data. *Nat Methods,* 2016, vol. 13, 581-583, https://doi.org/10.1038/nmeth.3869
- **MARIJON P. ; CHIKHI R. ; VARRÉ JS.** yacrd and fpa: upstream tools for long-read genome assembly. *Bioinformatics, https://doi.org/10.1093/bioinformatics/btaa262*
- **ABDULLA, M. ; MOHAMMED, N.** A Review on Inflammatory Bowel Diseases: Recent Molecular Pathophysiology Advances. Biologies. *Targets and Therapy,* 2022, vol. 16, 129-140
- **ACHARYA, K. ; KHANAL, S. ; PANTHA, K. ; AMATYA, N. ; DAVENPORT, R. J. ; WERNER, D.** A comparative assessment of conventional and molecular methods, including MinION nanopore sequencing, for surveying water quality. *Scientific reports,* 2019, vol. 9 (1), 1-11
- **AMMER-HERRMENAU, C. ; PFISTERER, N. ; VAN DEN BERG, T. ; GAVRILOVA, I. ; AMANZADA, A. ; SINGH, S. K. ; NEESSE, A.** Comprehensive wet-bench and bioinformatics workflow for complex microbiota using Oxford Nanopore technologies. *Msystems,* 2021, vol. 6 (4), e00750-21
- **BAUMGART, D. C. ; SANDBORN, W. J.** *Gastroenterology 2 Infl ammatory bowel disease: clinical aspects and established and evolving therapies,* 2007, vol. 369, www.thelancet.com

- **BEALE, D. J. ; BISSETT, A. ; NILSSON, S. ; BOSE, U. ; NELIS, J. L. D. ; NAHAR, A. ; VARDY, S.** Perturbation of the gut microbiome in wild-caught freshwater turtles (Emydura macquarii macquarii) exposed to elevated PFAS levels. *Science of The Total Environment,* 2022, 156324
- **BOLYEN, E. ; RIDEOUT, J. R. ; DILLON, M. R. ; BOKULICH, N. A. ; ABNET, C. C. ; AL-GHALITH, G. A. ; CAPORASO, J. G.** Reproducible, interactive, scalable and extensible microbiome data science using QIIME 2. *Nature biotechnology,* 2019, vol. 37 (8), 852-857
- **CALLAHAN, B. J. ; MCMURDIE, P. J. ; ROSEN, M. J. ; HAN, A. W. ; JOHNSON, A. J. A. ; HOLMES, S. P.** DADA2: High-resolution sample inference from Illumina amplicon data. *Nature methods,* 2016, vol. 13 (7), 581-583
- **CHAVAN, S. ; SARANGDHAR, V. ; VIGNESHWARAN, N.** Nanopore-based metagenomic analysis of the impact of nanoparticles on soil microbial communities. *Heliyon,* 2022, vol. 8 (6), e09693
- **COLE, J. R. ; WANG, Q. ; FISH, J. A. ; CHAI, B. ; MCGARRELL, D. M. ; SUN, Y. ; TIEDJE, J. M.** Ribosomal Database Project: data and tools for high throughput rRNA analysis. *Nucleic acids research,* 2014, vol. 42 (D1), D633-D642
- **DELAHAYE, C. ; NICOLAS, J.** Sequencing DNA with nanopores: Troubles and biases. *PLoS One,* 2021, vol. 16 (10), e0257521
- **DE SIQUEIRA, G. M. V. ; PEREIRA-DOS-SANTOS, F. M ; SILVA-ROCHA, R. ; GUAZZARONI, M. E.** Nanopore sequencing provides rapid and reliable insight into microbial profiles of intensive care units. *Frontiers in public health,* 2021, 1231
- **DRAGONI, G. ; INNOCENTI, T. ; GALLI, A.** Biomarkers of Inflammation in Inflammatory Bowel Disease: How Long before Abandoning Single-Marker Approaches?. *Digestive Diseases,* 2021, vol. 39, 190-203
- **EDGAR, R. C.** Search and clustering orders of magnitude faster than BLAST. *Bioinformatics,* 2010, vol. 26 (19), 2460-2461
- **EDGAR, R. C. ; HAAS, B. J. ; CLEMENTE, J. C. ; QUINCE, C. ; KNIGHT, R.** UCHIME improves sensitivity and speed of chimera detection. *Bioinformatics,* 2011, vol. 27 (16), 2194-2200

- **FUJIYOSHI, S. ; MUTO-FUJITA, A. ; MARUYAMA, F.** Evaluation of PCR conditions for characterizing bacterial communities with full-length 16S rRNA genes using a portable nanopore sequencer. *Scientific reports,* 2020, vol. 10 (1), 1-10
- **FU, Y ; CHEN, Q. ; XIONG, M. ; ZHAO, J. ; SHEN, S ; CHEN, L. ; LI, Y.** Clinical Performance of Nanopore Targeted Sequencing for Diagnosing Infectious Diseases. *Microbiology Spectrum,* 2022, vol. 10 (2), e00270-22
- **GEVERS, D. et al.** The treatment-naive microbiome in new-onset Crohn's disease. *Cell Host Microbe,* 2014, vol. 15, 382-392
- **HANAUER, S. B.** *Inflammatory Bowel Disease: Epidemiology, Pathogenesis, and Therapeutic Opportunities,* https://academic.oup.com/ibdjournal/article/12/suppl_1/S3/4676576
- **HEIKEMA, A. P. ; HORST-KREFT, D. ; BOERS, S. A. ; JANSEN, R. ; HILTEMANN, S. D. ; DE KONING, W. ; HAYS, J. P.** Comparison of Illumina versus nanopore 16S rRNA gene sequencing of the human nasal microbiota. *Genes,* 2020, vol. 11 (9), 1105
- **JANDA, J. M. ; ABBOTT, S. L.** 16S rRNA gene sequencing for bacterial identification in the diagnostic laboratory: pluses, perils, and pitfalls. *Journal of clinical microbiology,* 2007, vol. 45 (9), 2761-2764
- **JOHNSON, J. S. ; SPAKOWICZ, D. J. ; HONG, B. Y. ; PETERSEN, L. M. ; DEMKOWICZ, P. ; CHEN, L. ; WEINSTOCK, G. M.** Evaluation of 16S rRNA gene sequencing for species and strain-level microbiome analysis. *Nature communications,* 2019, vol. 10 (1), 1-11
- **KAPLAN, G. G.** The global burden of IBD: From 2015 to 2025. *Nature Reviews Gastroenterology and Hepatology,* 2015, vol. 12, 720-727, https://doi.org/10.1038/nrgastro.2015.150
- **KELSEN, J. ; BALDASSANO, R. N.** Inflammatory bowel disease: the difference between children and adults. *Inflamm Bowel Dis,* 2008, vol. 14 (2
- **KINOSHITA, Y. ; NIWA, H. ; UCHIDA-FUJII, E. ; NUKADA, T.** Establishment and assessment of an amplicon sequencing method targeting the 16S-ITS-23S rRNA operon for analysis of the equine gut microbiome. *Scientific reports,* 2021, vol. 11 (1), 1-12
- **KIM, M. ; OH, H. S. ; PARK, S. C. ; CHUN, J.** Towards a taxonomic coherence between average nucleotide identity and 16S rRNA gene sequence similarity for species demarcation of prokaryotes. *International journal of systematic and evolutionary microbiology,* 2014, vol. 64 (2), 346-351
- **KOZICH, J. J., WESTCOTT S. L., BAXTER N. T, HIGHLANDER S.K, SCHLOSS P.D.** Development of a dual-index sequencing strategy and curation pipeline for analyzing amplicon sequence data on the MiSeq Illumina sequencing platform. *Applied and environmental microbiology,* 2013, vol. 79 (17), 5112-5120
- **KUENZIG, M. E. et al.** Twenty-first Century Trends in the Global Epidemiology of Pediatric-Onset Inflammatory Bowel Disease: Systematic Review. *Gastroenterology,* 2022, vol. 162, 1147-1159
- **LI, X. ; KONG, P. ; DAUGHTREY, M. ; KOSTA, K. ; SCHIRMER, S. ; HOWLE, M. ; HONG, C.** Characterization of the Soil Bacterial Community from Selected Boxwood Gardens across the United States. *Microorganisms,* 2022, vol. 10 (8), 1514
- **LOW, L. ; FUENTES-UTRILLA, P. ; HODSON, J. ; O'NEIL, J. D. ; ROSSITER, A. E. ; BEGUM, G. ; RAUZ, S.** Evaluation of full-length nanopore 16S sequencing for detection of pathogens in microbial keratitis. *PeerJ,* 2021, vol. 9, e10778
- **MARTIN, M.** Cutadapt removes adapter sequences from high-throughput sequencing reads. *EMBnet. journal,* 2011, vol. 17 (1), 10-12
- **MATSUO, Y. ; KOMIYA, S. ; YASUMIZU, Y. ; YASUOKA, Y. ; MIZUSHIMA, K. ; TAKAGI, T. ; HIROTA, K.** Full-length 16S rRNA gene amplicon analysis of human gut microbiota using MinION™ nanopore sequencing confers species-level resolution. *BMC microbiology,* 2021, vol. 21 (1), 1-13
- **MOTTAWEA, W. et al.** Altered intestinal microbiota-host mitochondria crosstalk in new onset Crohn's disease. *Nat Commun,* 2016, 7
- **NYGAARD, A. B. ; TUNSJØ, H. S. ; MEISAL, R. ; CHARNOCK, C.** A preliminary study on the potential of Nanopore MinION and Illumina MiSeq 16S rRNA gene sequencing to characterize building-dust microbiomes. *Scientific Reports,* 2020, vol. 10 (1), 1-10
- **OBERLE, A. ; URBAN, L. ; FALCH-LEIS, S. ; ENNEMOSER, C. ; NAGAI, Y. ; ASHIKAWA, K. ; FEICHTINGER, M.** 16S rRNA long-read nanopore sequencing is feasible and reliable for endometrial microbiome analysis. *Reproductive Biomedicine Online,* 2021, vol. 42 (6), 1097-1107
- **OKSANEN J ; SIMPSON G ; BLANCHET F ; KINDT R ; LEGENDRE P ; MINCHIN P ; O'HARA R ; SOLYMOS P ; STEVENS M ; SZOECS E.** *vegan: Community Ecology Package_. R package version 2.6-2,* 2022, <https://CRAN.R-project.org/package=vegan>
- **OLBJORN, C. ; SMÅSTUEN, M. C. ; MOEN, A. E. F.** Targeted Analysis of the Gut Microbiome for Diagnosis, Prognosis and Treatment Individualization in Pediatric Inflammatory Bowel Disease. *Microorganisms,* 2022, 10

- **OMI, M. ; MATSUO, Y. ; ARAKI-SASAKI, K. ; OBA, S. ; YAMADA, H. ; HIROTA, K. ; TAKAHASHI, K.** 16S rRNA nanopore sequencing for the diagnosis of ocular infection: a feasibility study. *BMJ Open Ophthalmology,* 2022, vol. 7 (1), e000910
- **PARK, C. ; KIM, S. B. ; CHOI, S. H. ; KIM, S.** Comparison of 16S rRNA gene based microbial profiling using five next-generation sequencers and various primers. *Frontiers in Microbiology,* 2021, 12
- **PICHLER, M. ; COSKUN, Ö. K. ; ORTEGA-AR-BULÚ, A. S. ; CONCI, N. ; WÖRHEIDE, G. ; VAR-GAS, S. ; ORSI, W. D.** A 16S rRNA gene sequencing and analysis protocol for the Illumina MiniSeq platform. *Microbiologyopen,* 2018, vol. 7 (6), e00611
- **POLLOCK, J. ; GLENDINNING, L. ; WISED-CHANWET, T. ; WATSON, M.** The madness of microbiome: attempting to find consensus "best practice" for 16S microbiome studies. *Applied and environmental microbiology,* 2018, vol. 84 (7), e02627-17
- **QUAST, C. ; PRUESSE, E. ; YILMAZ, P. ; GERKEN, J. ; SCHWEER, T. ; YARZA, P. ; GLÖCKNER, F. O.** The SILVA ribosomal RNA gene database project: improved data processing and web-based tools. *Nucleic acids research,* 2012, vol. 41 (D1), D590-D596
- **ROGNES, T. ; FLOURI, T. ; NICHOLS, B. ; QUINCE, C. ; MAHÉ, F.** VSEARCH: a versatile open source tool for metagenomics. *PeerJ,* 2016, vol. 4, e2584
- **ROSEN, M. J. ; DHAWAN, A. ; SAEED, S. A.** Inflammatory bowel disease in children and adolescents. *JAMA Pediatrics,* 2015, vol. 169, 1053-1060, https://doi.org/10.1001/jamapediatrics.2015.1982
- **ROZAS, M. ; BRILLET, F. ; CALLEWAERT, C. ; PA-ETZOLD, B.** MinION™ Nanopore Sequencing of Skin Microbiome 16S and 16S-23S rRNA Gene Amplicons. *Frontiers in cellular and infection microbiology,* 2022, 1317
- **RUSSEL, J.** *Russel88/MicEco: v0.9.15 (v0.9.15), Zenodo,* 2021, https://doi.org/10.5281/zenodo.4733747
- **SAUER, C. G. ; KUGATHASAN, S.** Pediatric Inflammatory Bowel Disease: Highlighting Pediatric Differences in IBD. *Medical Clinics of North America,* 2010, vol. 94, 35-52, https://doi.org/10.1016/j.mcna.2009.10.002
- **SCHLOSS, P. D. ; WESTCOTT, S. L. ; RYABIN, T. ; HALL, J. R. ; HARTMANN, M. ; HOLLISTER, E. B. ; WEBER, C. F.** Introducing mothur: open-source, platform-independent, community-supported software for describing and comparing microbial communities. *Applied and environmental microbiology,* 2009, vol. 75 (23), 7537-7541
- **SHIN, J. ; LEE, S. ; GO, M. J. ; LEE, S. Y. ; KIM, S. C. ; LEE, C. H. ; CHO, B. K.** Analysis of the mouse gut microbiome using full-length 16S rRNA amplicon sequencing. *Scientific reports,* 2016, vol. 6 (1), 1-10
- **SILA, S. et al.** Altered Gut Microbiota Is Present in Newly Diagnosed Pediatric Patients With Inflammatory Bowel Disease. *J Pediatr Gastroenterol Nutr,* 2020, vol. 70, 497-502
- **SILANGCRUZ, K. et al.** Impact of the World Inflammatory Bowel Disease Day and Crohn's and Colitis Awareness Week on Population Interest Between 2016 and 2020: Google Trends Analysis. *JMIR Infodemiology,* 2021, vol. 1, e32856
- **STAHL-ROMMEL, S. ; JAIN, M. ; NGUYEN, H. N. ; ARNOLD, R. R. ; AUNON-CHANCELLOR, S. M. ; SHARP, G. M. ; CASTRO-WALLACE, S. L.** Real-time culture-independent microbial profiling onboard the international space station using nanopore sequencing. *Genes,* 2021, vol. 12 (1), 106
- **STEVENS, B. ; CREED, T. ; REARDON, C. ; MAN-TER, D.** *Comparison of Oxford Nanopore Technologies and Illumina MiSeq sequencing with mock communities and agricultural soil.,* 2022
- **URBAN, L. ; HOLZER, A. ; BARONAS, J. J. ; HALL, M. B. ; BRAEUNINGER-WEIMER, P. ; SCHERM, M. J. ; STAMMNITZ, M. R.** Freshwater monitoring by nanopore sequencing. *Elife,* 2021, 10
- **VAN DER LOOS, L. M. ; D'HONDT, S. ; WILLEMS, A. ; DE CLERCK, O.** Characterizing algal microbiomes using long-read nanopore sequencing. *Algal Research,* 2021, vol. 59, 102456
- **WEI, P. L. ; HUNG, C. S. ; KAO, Y. W. ; LIN, Y. C. ; LEE, C. Y. ; CHANG, T. H. ; LIN, J. C.** Characterization of fecal microbiota with clinical specimen using long-read and short-read sequencing platform. *International journal of molecular sciences,* 2020, vol. 21 (19), 7110
- **WINAND, R. ; BOGAERTS, B. ; HOFFMAN, S. ; LEFEVRE, L. ; DELVOYE, M. ; VAN BRAEKEL, J. ; VANNESTE, K.** Targeting the 16s rRNA gene for bacterial identification in complex mixed samples: Comparative evaluation of second (illumina) and third (oxford nanopore technologies) generation sequencing technologies. *International journal of molecular sciences,* 2019, vol. 21 (1), 298
- **YU, Y. R. ; RODRIGUEZ, J. R.** Clinical presentation of Crohn's, ulcerative colitis, and indeterminate colitis: Symptoms, extraintestinal manifestations, and disease phenotypes. *Semin Pediatr Surg,* 2017, vol. 26, 349-355
- **ZHAO, M. ; GÖNCZI, L ; LAKATOS, P. L. ; BUR-ISCH, J.** The Burden of Inflammatory Bowel Disease in Europe in 2020. *Journal of Crohn's and Colitis,* 2021, vol. 15, 1573-1587, https://doi.org/10.1093/ecco-jcc/jjab029